# EUROPEAN PATENT APPLICATION

(11) **EP 3 903 579 A1**
(43) Date of publication of application: **03.11.2021**
(21) Application number: 19905670.6
(22) Date of filing: 27.12.2019
(51) Int. Cl.: A01K 67/027, C12N 5/071

(54) **SYSTEMIC SCLEROSIS DISEASE MODEL AND USE THEREOF**

(30) Priority: 28.12.2018 KR 20180172694
(71) Applicant: YiPSCELL Inc., Seoul 06579 (KR)
(72) Inventor: JU, Ji Hyeon, Seoul 06602 (KR); KIM, Ye Na, Seongnam-si, Gyeonggi-do 13128 (KR)
(74) Representative: SONN Patentanwälte OG
(86) International application number: PCT/KR2019/018654
(87) International publication number: WO 2020/139041

(57) **Abstract**

The present invention relates to a production method of a systemic sclerosis disease model using keratinocytes and fibroblasts differentiated from induced pluripotent stem cells derived from patients with systemic sclerosis, a systemic sclerosis disease model produced thereby, and a method for screening a therapeutic agent for systemic sclerosis using the same.

The systemic sclerosis disease model produced by the above production method can be effectively used for preventing or treating systemic sclerosis.

## Description

### CROSS-REFERENCE TO RELATED APPLICATION

This application claims priority to and the benefit of Korean Patent Application No. 10-2018-0172694, filed on December 28, 2018, the disclosure of which is incorporated herein by reference in its entirety.

### Technical Field

The present invention relates to a method for producing a systemic sclerosis disease model using keratinocytes and/or fibroblasts differentiated from induced pluripotent stem cells derived from patients with systemic sclerosis, a systemic sclerosis disease model produced thereby, and a method for screening a therapeutic agent for systemic sclerosis using the same.

### Background Art

Systemic sclerosis (SSc) is a disease caused by excessive production of connective tissue components such as collagen and the like by activated fibroblasts. SSc causes changes in the skin, as well as connective tissue such blood vessels, gastrointestinal system, lungs, kidneys, muscle joints, and the like, resulting in defects in the function thereof. Depending on the site of the disease, SSc is mainly classified into a limited type in which skin hardening occurs only in the hands below the elbow, the feet below the knee, and the face, and a diffuse type in which changes of skin hardening occur in more areas than the above, that is, skin above the elbow and above the knee and the body, accompanied by invasion of internal organs such as kidneys, lungs, and the like.

Induced pluripotent stem cells (iPS cells) refer to cells having pluripotency obtained by dedifferentiation from differentiated cells, such as somatic cells, and it is possible to be differentiated into various organ cells. Since induced pluripotent stem cells can be obtained by reprogramming cells due to dedifferentiation-inducing factors, it is possible to generate a patient immune-compatible pluripotent cell line without somatic cell transfer. All of induced pluripotent stem cells have pluripotency with the ability to produce all of the cells of our body, and have the ability of self-renewal to infinitely create cells that resemble themselves. Human embryonic stem cells also show pluripotency, but due to ethical problems, the scope of research and application is limited, whereas induced pluripotent stem cells have an advantage of being free from ethical problems because human somatic cells can be used.

Induced pluripotent stem cells (iPSC) are expected to be able to fundamentally solve incurable diseases that are difficult to treat with drugs or surgery by replacing damaged cells, tissues, or organs in the future due to the pluripotency thereof, and furthermore, these are expected to be utilized in various fields of life science ranging from developing new drugs, studying disease mechanisms, and conducting developmental studies. However, the induced pluripotent stem cell technology has remained at a basic stage, and it is focused on developing a technique for producing and establishing induced pluripotent stem cells mainly by inducing dedifferentiation.

Meanwhile, the establishment of induced pluripotent stem cells using patient-derived cells and the development of a drug screening system based thereon have been attempted by several researchers over the past decade since the discovery of the method of inducing induced pluripotent stem cells by Professor Yamanaka in 2006. In diseases mainly caused by factors such as gene (DNA) abnormalities and the like, many of these disease organoids have been tried and applied to the screening of drugs. In the case of a disease caused by gene abnormality, when it is dedifferentiated from somatic cells of the patient and differentiated back into a specific cell, the corresponding gene is certainly copied as it is, and the gene causing the disease is also transferred as it is. As a result, the possibility is very high that the phenotype of the disease appears in re-differentiated cells. However, in the case of systemic sclerosis, many genetic studies have been conducted on patients, but as far as it is known, it is not a disease caused by a defect in the gene, and the exact mechanism of pathogenesis has not been revealed. In addition, there has been no report on techniques for producing a systemic sclerosis disease model in which the human immune system is implemented using human induced pluripotent stem cells, or selecting a treatment method or drug suitable for individuals by utilizing such a disease model.

Under these backgrounds, the present inventors produced a systemic sclerosis disease model, and as a result of intensive research efforts to develop a method for screening a therapeutic agent of systemic sclerosis using the same and a prophylactic or therapeutic agent of systemic sclerosis, keratinocytes and fibroblasts were successfully differentiated from induced pluripotent stem cells derived from patients with systemic sclerosis, and a new systemic sclerosis skin model was constructed using these two cells in 3D culture. In addition, the present invention was completed by confirming disease-specific markers of systemic sclerosis in the cell and skin model by various methods *in vitro.*

### Disclosure

### Technical Problem

An object of the present invention is to provide a method for producing a systemic sclerosis disease model, including differentiating induced pluripotent stem cells (iPSC) derived from patients with systemic sclerosis into keratinocytes or fibroblasts.

Another object of the present invention is to provide a systemic sclerosis disease model produced by the above production method.

Still another object of the present invention is to provide a method for differentiating patient-derived induced pluripotent stem cells into keratinocytes.

Still another object of the present invention is to provide a method for differentiating patient-derived pluripotent stem cells into fibroblasts in which a patient's disease phenotype is simulated.

Still another object of the present invention is to provide a 3D skin organoid including keratinocytes produced by the above method; and fibroblasts produced by the above method.

Still another object of the present invention is to provide fibroblasts derived from induced pluripotent stem cells with increased expression of a marker of fibrosis compared to normal cells.

Still another object of the present invention is to provide a method for screening an antifibrotic drug including a therapeutic agent for systemic sclerosis using the systemic sclerosis disease model.

Still another object of the present invention relates to a therapeutic agent for systemic sclerosis screened by the above method.

### Technical Solution

An aspect of the present invention for achieving the above objects relates to a method for producing a systemic sclerosis disease model, including a step of differentiating induced pluripotent stem cells (iPSC) derived from patients with systemic sclerosis (SSc) into keratinocytes or fibroblasts; and a step of forming a 3D skin organoid by three-dimensionally culturing the keratinocytes and/or fibroblasts.

In addition, the present invention relates to a method for producing a systemic sclerosis disease model, including a step of differentiating induced pluripotent stem cells (iPSC) derived from patients with systemic sclerosis (SSc) into keratinocytes or fibroblasts; a step of forming a 3D skin organoid by three-dimensionally culturing the keratinocytes or fibroblasts; and a step of transplanting the 3D skin organoid into the skin tissue of a mouse,

In addition, the present invention relates to a systemic sclerosis disease model, produced by the above methods.

Another aspect of the present invention relates to a method for differentiating induced pluripotent stem cells into keratinocytes, including a step of primarily culturing an embryonic body (EB) of induced pluripotent stem cells (iPSC) derived from patients with systemic sclerosis (SSc) in Differentiation Culture Medium 1 including DMEM/F 12 for 3 to 10 days; a step of secondarily culturing for 2 to 10 days by replacing the culture medium with Differentiation Culture Medium 2 including a defined keratinocyte serum-free medium (DKSFM); and a step of tertiarily culturing for 5 to 60 days by replacing the secondary culture medium with Differentiation Culture Medium 3 including DKSFM and a keratinocyte serum-free medium (KSFM).

In the differentiation method, the primary culture period may be 7 to 9 days, the secondary culture period may be 3 to 6 days, and the tertiary culture period may be 15 to 25 days.

In addition, the present invention relates to a method for differentiating iPSC into fibroblasts, including a step of culturing an embryonic body (EB) of induced pluripotent stem cells (iPSC) derived from patients with systemic sclerosis (SSc) in Differentiation Culture Medium 1 including DMEM/F12 and epidermal growth factor (EGF) for 1 to 7 days; a step of maintaining for 1 to 7 days by adding BMP4 to the Differentiation Culture Medium 1; a step of secondarily culturing for 5 to 15 days by replacing the culture medium with Differentiation Culture medium 2 including DMEM/F12 thereafter; and a step of tertiarily culturing for 5 to 20 days by replacing the culture medium with Differentiation Culture Medium 1 thereafter.

In the differentiation method, the primary culture period may be 2 to 5 days, the secondary culture period may be 5 to 10 days, and the tertiary culture period may be 5 to 15 days. In addition, it may include additionally culturing by placing the cultured cells after tertiarily culturing on a collagen 1-coated plate.

The differentiation method may further include additionally culturing for 5 days or more by placing on a collagen 1-coated plate.

Another aspect of the present invention relates to a 3D skin organoid, including keratinocytes produced by the above method; and fibroblasts produced by the above method. In the 3D skin organoid, in cells of the organoid, the accumulation of collagen may be increased, and the expression of α-smooth muscle actin (a-SMA) may be increased, compared to normal cells.

Still another aspect of the present invention relates to a fibroblast derived from induced pluripotent stem cells, wherein the fibroblast is a fibroblast derived from induced pluripotent stem cells from patients with systemic sclerosis in which the expression of a fibrosis marker is increased compared to a fibroblast derived from normal induced pluripotent stem cells, and wherein the fibrosis marker includes one or more selected from the group consisting of COL1A1, COL1A2, COL3A1, ACTA2, and vimentin. The induced pluripotent cells may be reprogrammed cells from cells from patients with systemic sclerosis. In the fibroblast, the accumulation of collagen may be increased, and the expression of α-smooth muscle actin (a-SMA) may be increased, compared to a fibroblast derived from normal induced pluripotent stem cells.

Still another aspect of the present invention relates to a systemic sclerosis disease model, including the 3D skin organoid or fibroblast. Herein, the disease model may be a mouse.

In addition, the present invention relates to a systemic sclerosis disease model, including cells or a 3D cell aggregate formed by culturing induced pluripotent stem cells (iPSC) derived from patients with systemic sclerosis (SSc) and differentiating into skin cells, wherein the cells or cell aggregate exhibit a pathological property of systemic sclerosis, and wherein the pathological property of cells exhibits increased cell proliferation and the increased production and accumulation of fibrosis markers and collagen and the like, compared to normal induced pluripotent stem cells.

As used herein, the term "induced pluripotent stem cells (iPSC) derived from patients with systemic sclerosis" refers to cells induced to have pluripotency through the process of reprogramming from peripheral blood cells (PBMC) in patients with systemic sclerosis.

As used herein, the term "reprogramming " refers to a process that may finally be restored or converted to a state with a new type of differentiation potential from differentiated cells that exist in different modes, such as cells that do not have differentiation capacity, cells with a certain ability to differentiate, or the like. In addition, in the present invention, the reprogramming may be used in the same meaning as dedifferentiation. The reprogramming mechanism of these cells means establishing a different set of epigenetic marks after the epigenetic marks in the nucleus (the DNA state associated with causing genetic changes in function without changes in the nucleotide sequence) are deleted. However, while multicellular organisms differentiate and grow, different cells and tissues acquire different gene expression programs.

The reprogramming is not limited as long as it is possible to make induced pluripotent stem cells from PBMC of patients with systemic sclerosis, and a known method in the art may be used.

Specifically, in the present invention, the artificial reprogramming process may be performed by the introduction of a virus-mediated or non-insertable non-viral vector using a non-insertable virus, a non-virus-mediated reprogramming factor using a protein, a cell extract, and the like, or may include a reprogramming process by stem cell extracts, compounds, and the like. Induced pluripotent stem cells have almost the same characteristics as embryonic stem cells. Specifically, it may show a similar cell shape, have similar gene and protein expression patterns and pluripotent differentiation ability *in vitro* and *in vivo,* and form teratomas, and when it is inserted into blastocysts of mice, it may form chimera mice, and germline transmission of genes is possible. The induced pluripotent stem cells of the present invention may be derived from humans, monkeys, pigs, horses, cows, sheep, dogs, cats, mice, rabbits, or the like, and may be specifically derived from humans.

As used herein, the term "reprogramming factor" is a substance that induces finally differentiated cells to be reprogrammed into pluripotent stem cells having a new type of differentiation potential. The reprogramming factor may include, without limitation, any substance that induces reprogramming of the finally differentiated cell, and may be selected according to the type of cells to be differentiated. Specifically, the reprogramming factor may be one or more proteins selected from the group consisting of Oct4, Sox2, Klf4, c-Myc, Nanog, Lin-28, and Rex1, or a nucleic acid molecule encoding the protein, but is not limited thereto.

As used herein, the term "nucleic acid molecule encoding a protein" may be in a form operably linked to a promoter and the like so as to express the corresponding protein itself when delivered into a cell.

As used herein, the term "differentiation" refers to a phenomenon in which cells divide and proliferate, and the structure or function of cells is specialized while the entire individual is growing. That is, it refers to a process in which cells, tissues, and the like of an organism are transformed into a suitable form and function in order to perform a role given to each, for example, a process in which pluripotent stem cells such as embryonic stem cells are transformed into ectodermal, mesodermal, and endoderm cells, as well as a process in which hematopoietic stem cells are transformed into red blood cells, white blood cells, platelets, and the like, that is, progenitor cells expressing a specific differentiation trait may be included in differentiation.

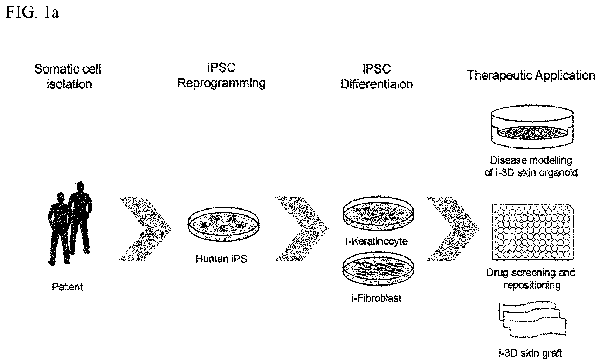

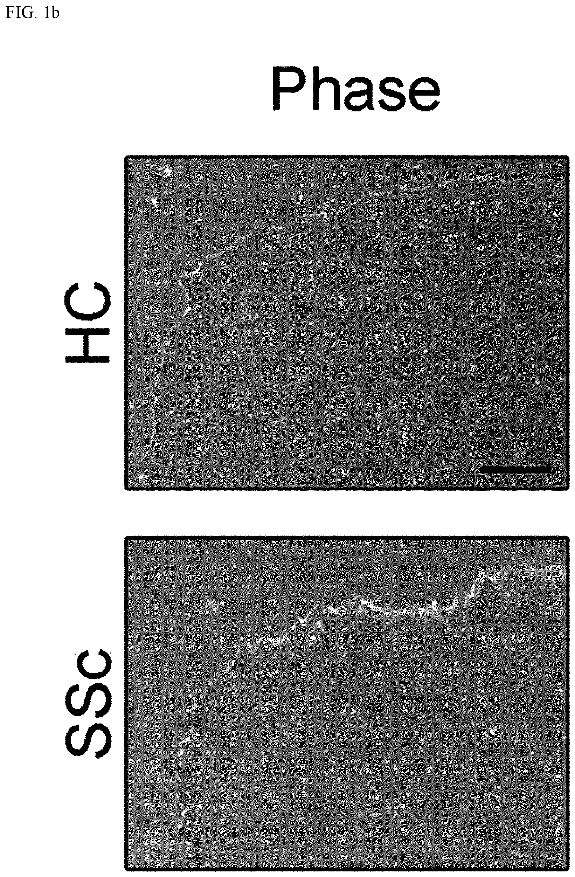

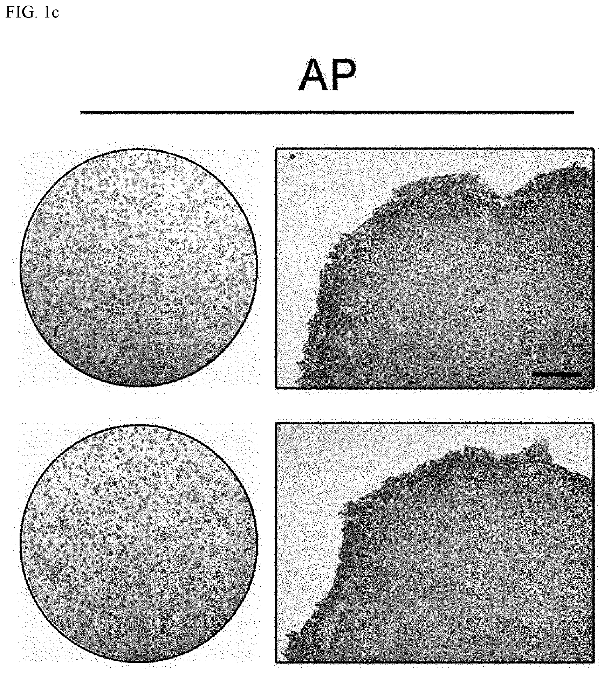

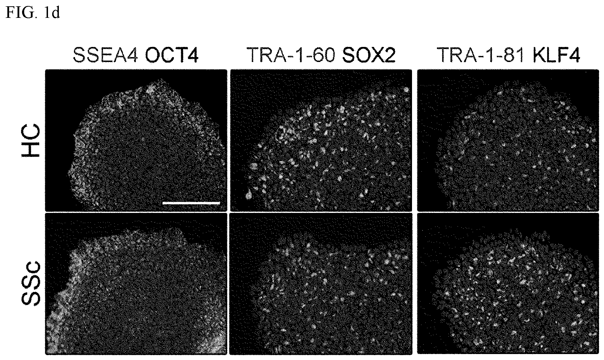

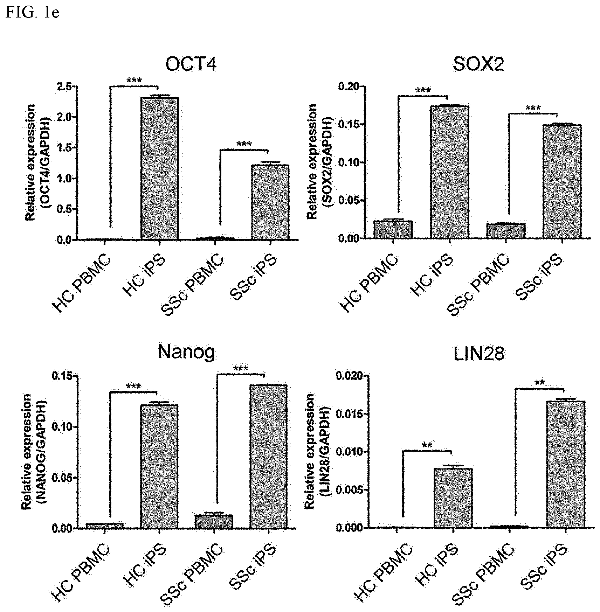

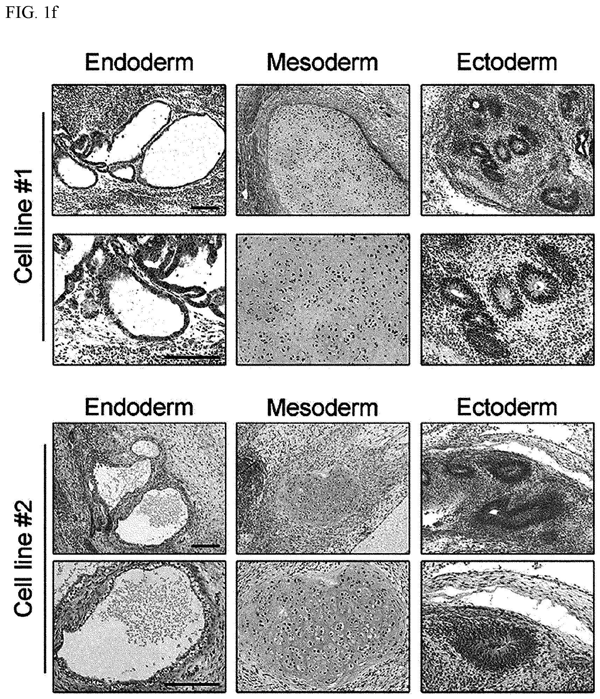

In a specific exemplary embodiment of the present invention, induced pluripotent stem cells (iPSC) were produced through reprogramming from PBMC of the blood of patients with systemic sclerosis (FIG. 1).

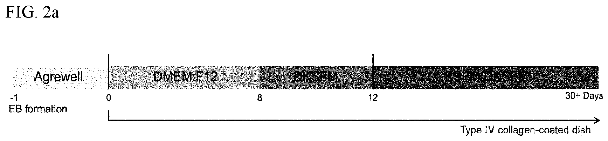

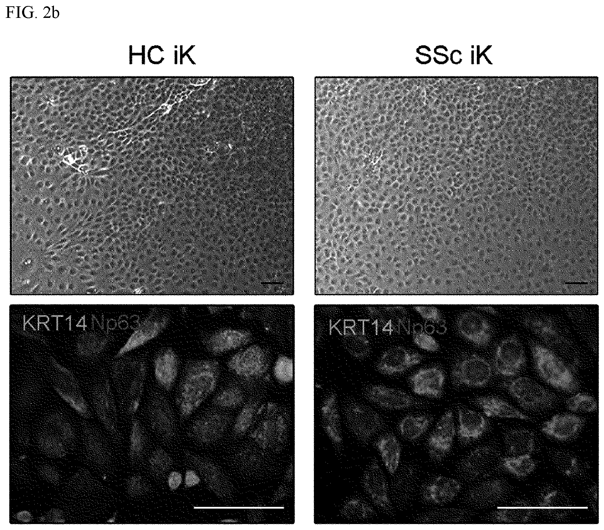

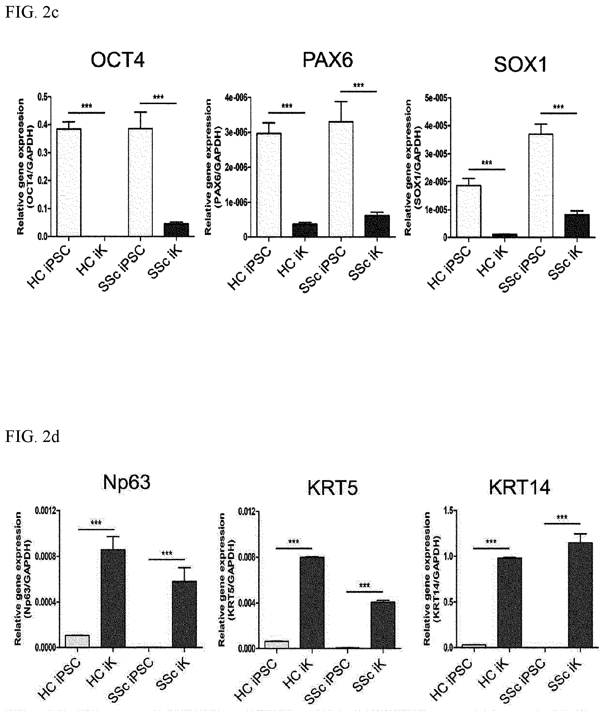

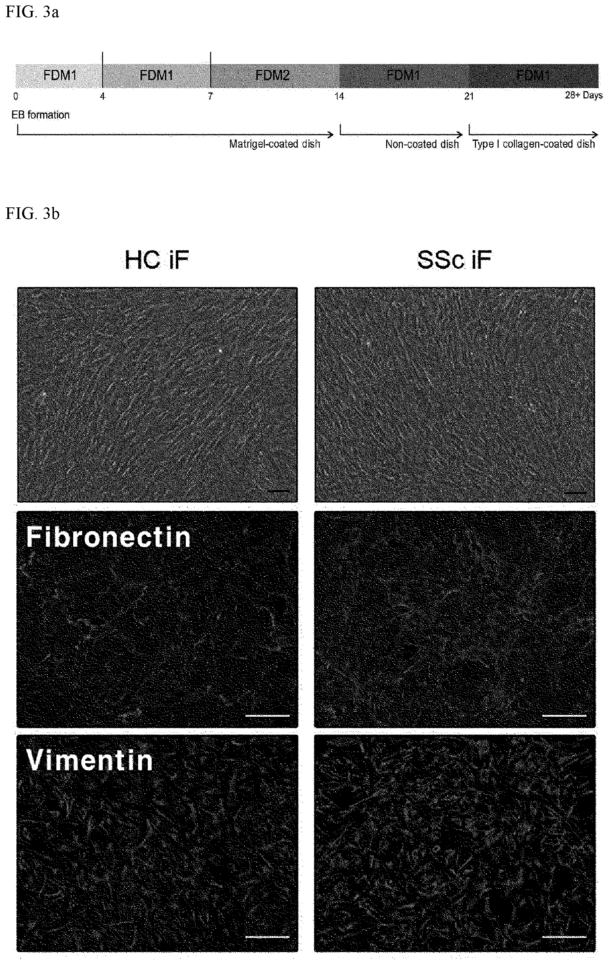

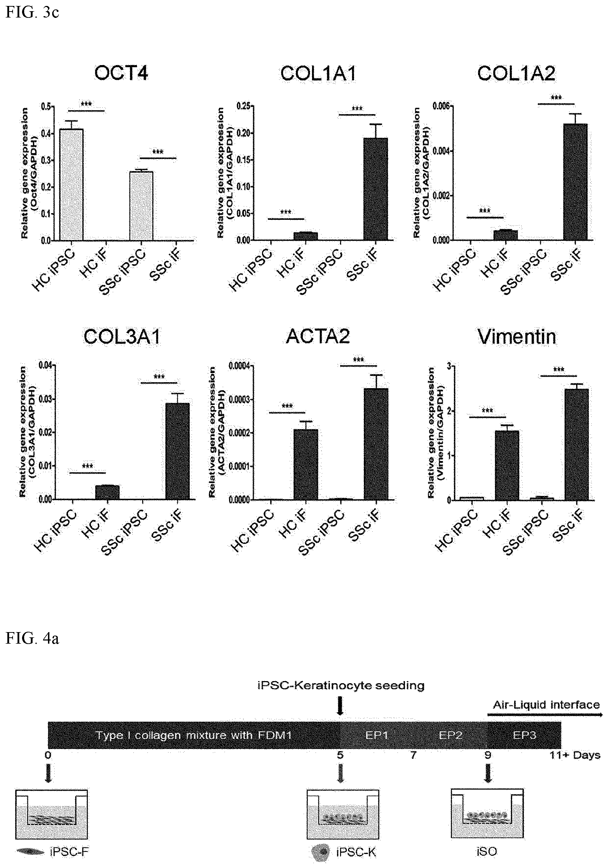

In addition, in a specific exemplary embodiment of the present invention, in order to prepare keratinocytes and fibroblasts having a potential to reproduce the phenotype of systemic sclerosis-specific disease, induced pluripotent stem cells derived from patients with systemic sclerosis were differentiated into keratinocytes or fibroblasts, respectively. It was found that keratinocytes and fibroblasts derived from induced pluripotent stem cells were produced by confirming the expressions of the markers of keratinocytes or fibroblasts, the markers of proteins and cell surfaces, and the like (FIGS. 2 and 3).

Specifically, the production method of the present invention may include differentiating induced pluripotent stem cells derived from patients with systemic sclerosis into keratinocytes or fibroblasts, and producing a 3D skin organoid by three-dimensionally culturing the keratinocytes and fibroblasts.

As used herein, the term "3D organoid" refers to a cell mass having a 3D space structure, and refers to a reduced and simplified version of an organ produced through an artificial culturing process that is not collected and obtained from animals and the like. The origin of the cells constituting the same is not limited. An organoid may be derived from tissue, embryonic stem cells, or induced pluripotent stem cells, and may be three-dimensionally cultured due to their ability to self-renew and differentiate. The organoid may have an environment that is allowed to interact with the surrounding environment during the cell growth process. Accordingly, in the present invention, the "3D skin organoid" may be an excellent model for observing the development and treatment processes of a therapeutic agent for a disease by almost simulating a skin layer that actually interacts *in vivo.*

In the present invention, the 3D skin organoid produced by three-dimensionally culturing keratinocytes and fibroblasts derived from induced pluripotent stem cells from patients with systemic sclerosis may overcome spatial and temporal limitations in investigating the accumulation and decomposition processes of collagen related to systemic sclerosis that occurs when the existing 2D cell culture system is used. It is easy to confirm whether collagen is accumulated and the like by staining, and furthermore, it has an advantage of being able to recreate a systemic sclerosis disease model similarly and precisely compared to the environment in the human body by creating a humanized mouse model by additionally transplanting the same in the mouse skin.

Therefore, the 3D skin organoid or the humanized animal model into which 3D skin organoid is transplanted according to the present invention may be used as a systemic sclerosis disease model.

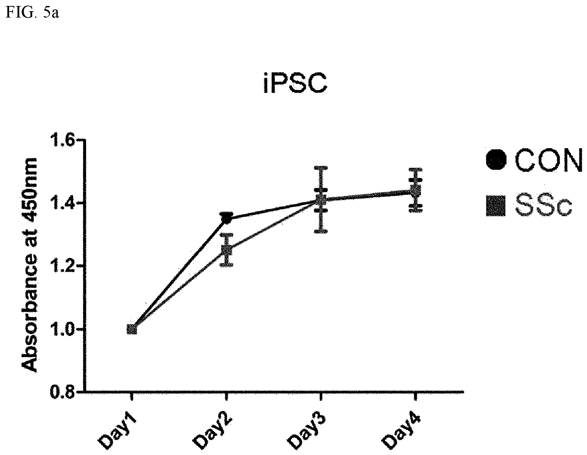

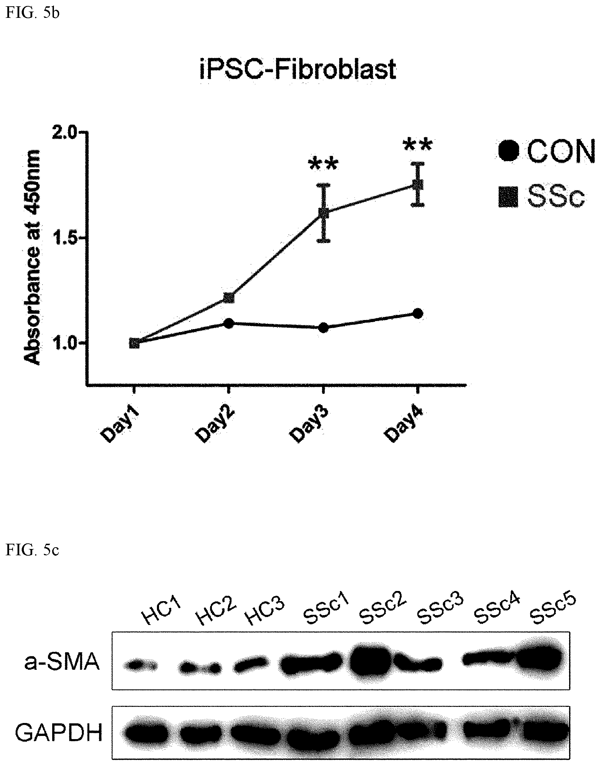

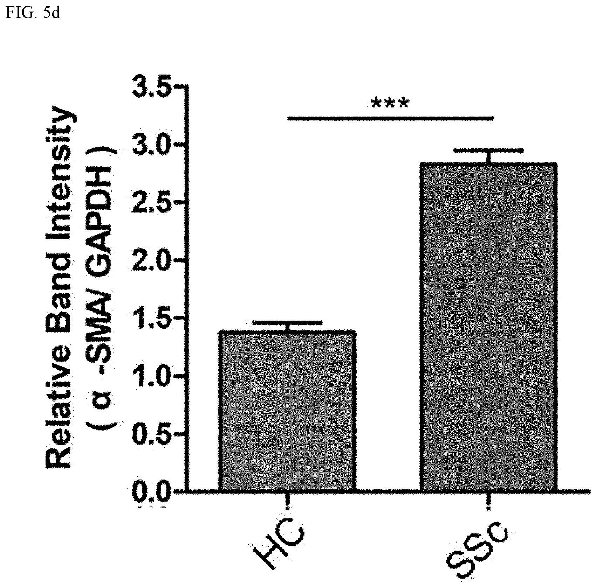

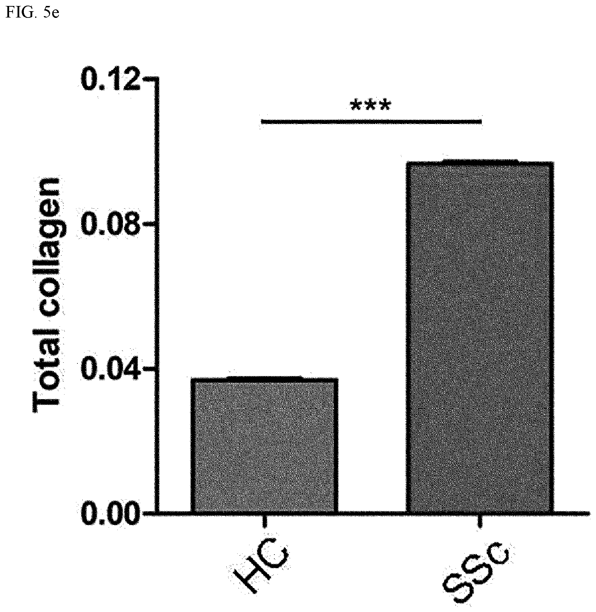

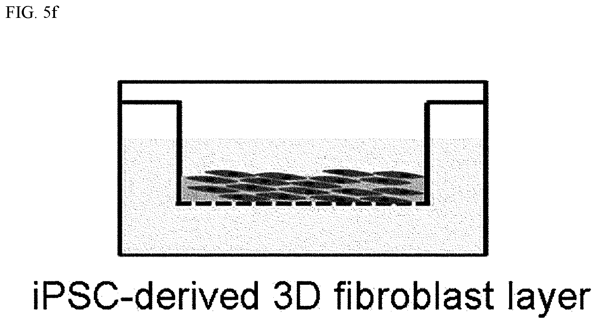

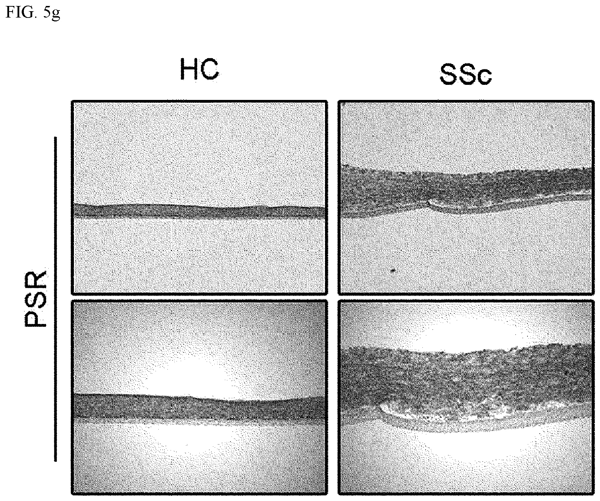

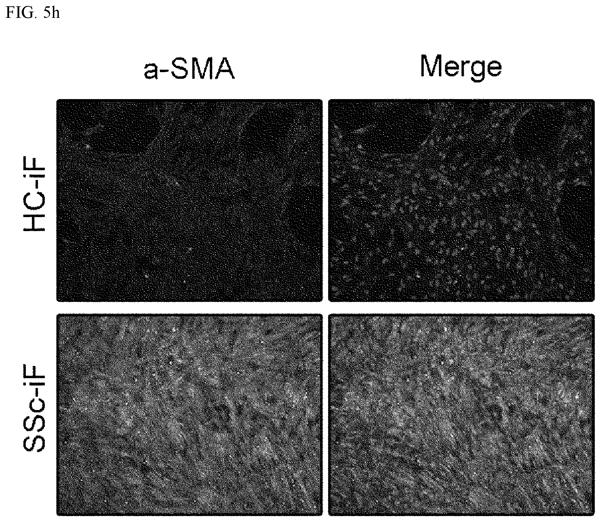

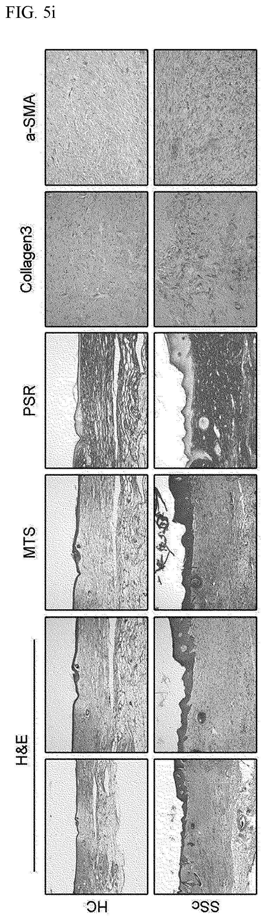

In a specific exemplary embodiment of the present invention, it was confirmed that the fibroblasts or 3D skin organoids produced through the above process had higher expression levels of α-SMA and collagen, which are fibroblast markers, compared to fibroblasts derived from induced pluripotent stem cells of normal people, and the thickness of the 3D fibroblast cell layer was increased compared to the normal. Furthermore, when the 3D skin organoid (iSO) of the present invention was transplanted into mice, it was confirmed that the thickness of the skin tissue was increased and the expressions of collagen and α-SMA were increased compared to mice transplanted with normal iSO (FIG. 5). Through this, it was found that the fibroblasts derived from induced pluripotent stem cells from patients, the 3D skin organoids, or the humanized animal model transplanted with the same according to the present invention may be used as new disease models for the development of a disease mechanism and a new therapeutic agent.

In addition, still another aspect of the present invention relates to a method for screening an antifibrotic drug including a therapeutic agent for systemic sclerosis, the method including (a) producing a 3D skin organoid by differentiating induced pluripotent stem cells (iPSC) derived from patients with systemic sclerosis (SSc) into keratinocytes or fibroblasts to three-dimensionally culture the keratinocytes and fibroblasts; (b) treating the 3D skin organoid with a test agent that inhibits collagen accumulation; (c) measuring the expression level of α-smooth muscle actin (a-SMA) or mRNA of a gene thereof in the 3D skin organoid treated with the test agent; and (d) determining the test agent as a therapeutic agent for systemic sclerosis, when the expression level of α-SMA or mRNA of a gene thereof measured in Step (c) is decreased compared to a 3D skin organoid not treated with a test agent.

In addition, the present invention relates to a method for screening an antifibrotic drug including a therapeutic agent for systemic sclerosis, the method including (a) producing a 3D skin organoid by differentiating induced pluripotent stem cells (iPSC) derived from patients with systemic sclerosis (SSc) into keratinocytes or fibroblasts to three-dimensionally culture the keratinocytes and fibroblasts; (b) treating the 3D skin organoid with a test agent that inhibits collagen accumulation; (c) measuring the level of collagen accumulation in the 3D skin organoid treated with the test agent; and (d) determining the test agent as a therapeutic agent for systemic sclerosis, when the level of collagen accumulation measured in Step (c) is decreased compared to a 3D skin organoid not treated with a test agent.

In addition, the present invention relates to a method for screening an antifibrotic drug including a therapeutic agent for systemic sclerosis, the method including (a) differentiating induced pluripotent stem cells (iPSC) derived from patients with systemic sclerosis (SSc) into fibroblasts; (b) treating the fibroblasts differentiated in Step (a) with a test agent that inhibits collagen accumulation; (c) measuring the expression level of α-smooth muscle actin (a-SMA) or mRNA of a gene thereof in the fibroblasts treated with the test agent; and (d) determining the test agent as a therapeutic agent for systemic sclerosis, when the expression level of α-SMA or mRNA of a gene thereof measured in Step (c) is decreased. compared to fibroblasts not treated with a test agent.

In addition, the present invention relates to a method for screening an antifibrotic drug including a therapeutic agent for systemic sclerosis, the method including (a) differentiating induced pluripotent stem cells (iPSC) derived from patients with systemic sclerosis (SSc) into fibroblasts; (b) treating the fibroblasts differentiated in Step (a) with a test agent that inhibits collagen accumulation; (c) measuring the level of collagen accumulation in the fibroblasts treated with the test agent; and (d) determining the test agent as a therapeutic agent for systemic sclerosis, when the level of collagen accumulation measured in Step (c) is decreased, compared to fibroblasts not treated with a test agent.

In addition, the present invention relates to an antifibrotic drug including a therapeutic agent for systemic sclerosis screened by the above methods.

As used herein, the term "collagen accumulation" refers to an abnormal accumulation of a collagen matrix following injury or inflammation that changes the structure and function of various tissues. Although this occurs naturally in organs and tissues under normal circumstances, it may occur excessively and may accompany or cause disease. Most etiology of systemic sclerosis includes excessive accumulation of a collagen matrix replacing normal tissue.

As used herein, the term "test agent" includes any substance, molecule, element, compound, entity, or combinations thereof. For example, while not limited thereto, it may include proteins, polypeptides, small organic molecules, polysaccharides, polynucleotides, and the like. In addition, it may also be a natural product, a synthetic compound, or a combination of two or more substances.

In fibroblasts, keratinocytes, or 3D skin organoids which are the systemic sclerosis disease models of the present invention, the expression or activity of α-SMA is reduced, and fibrosis is inhibited when collagen accumulation decreases. Therefore, an agent that reduces the expression of α-SMA or the expression level of mRNA of a gene thereof in fibroblasts, keratinocytes, or 3D skin organoids of the present invention and inhibits collagen accumulation may be used as an agent for the prevention or treatment of systemic sclerosis.

As used herein, the term "measurement of the expression level of protein" refers to a process of determining the expression level of α-SMA that affects collagen accumulation in fibrotic diseases, and it measures the amount of protein. Specifically, it may be an antibody or aptamer that specifically binds to a protein expressed from the α-SMA gene, but is not limited thereto. If such an antibody is a polyclonal antibody or a monoclonal antibody or has an antigen-binding property, fragments of the antibody are also included in the antibody of the present invention. Furthermore, the antibody of the present invention includes a special antibody such as a humanized antibody, a human antibody, and the like, and in addition to novel antibodies, antibodies already known in the art may also be included. The antibody includes not only a complete form having a full length of two heavy chains and two light chains, but also a functional fragment of an antibody molecule, as long as it has the characteristic of binding to specifically recognize a protein expressed from the α-SMA gene. The functional fragment of an antibody molecule refers to a fragment having at least an antigen-binding function, and may be Fab, F (ab'), F (ab')2, and Fv, but is not limited thereto.

For the purpose of the present invention, for the measurement of the expression level of protein, there are protein chip analysis, immunoassay, ligand binding assay, matrix desorption/ionization time of flight mass spectrometry (MALDI-TOF) analysis, surface enhanced laser desorption/ionization time of flight mass spectrometry (SELDI-TOF) analysis, radioimmunoassay, radial immunodiffusion assay, Ouchterlony immunodiffusion, rocket immunoelectrophoresis, tissue immunostaining, complement fixation analysis, 2D electrophoresis analysis, liquid chromatography-mass spectrometry (LCMS), liquid chromatography-mass spectrometry/mass spectrometry (LC-MS/MS), western blot, enzyme linked immunosorbent assay (ELISA), and the like, but the present invention is not limited thereto.

As used herein, the term "measurement of the expression level of mRNA" is a process of determining the expression level of the gene of α-SMA affecting collagen accumulation in systemic sclerosis, and it measures the amount of mRNA. Analysis methods for this include reverse transcriptase polymerase chain reaction (RT-PCR), competitive reverse transcriptase polymerase chain reaction (competitive RT-PCR), real-time reverse transcriptase polymerase chain reaction (real-time RT-PCR), RNase protection assay (RPA), Northern blotting, DNA chips, and the like, but the present invention is not limited thereto.

The agent for measuring the mRNA level of the gene may specifically include a primer pair, a probe, or an antisense nucleotide that specifically binds to the α-SMA gene, and since the nucleic acid information of the genes is published in GeneBank and the like, those skilled in the art may design primers or antisense nucleotides that specifically amplify specific regions of these genes based on the above sequences.

In a specific exemplary embodiment of the present invention, it was confirmed that the expression of total collagen and the expressions of COL1A1 and ACTA2 were reduced after treating with pirfenidone and nintedanib, which are drugs approved as therapeutic agents of pulmonary fibrosis. Through this, the effectiveness of the actual clinical drug was demonstrated, and the screening platform through the establishment of an induced pluripotent stem cell-derived fibrosis model was verified.

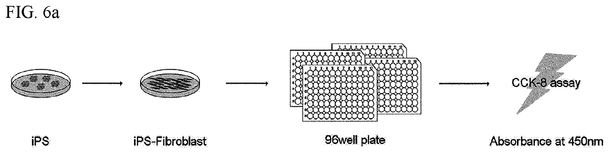

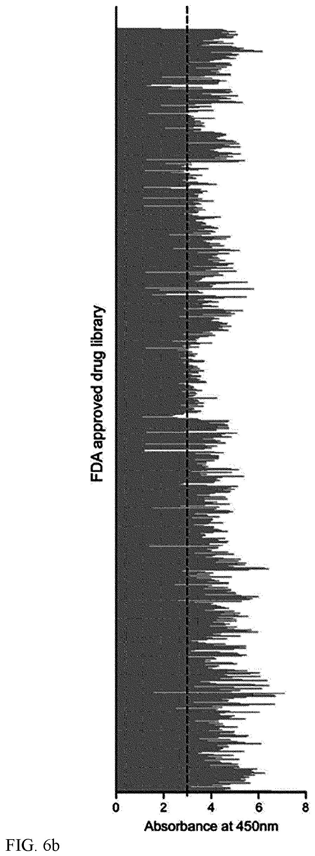

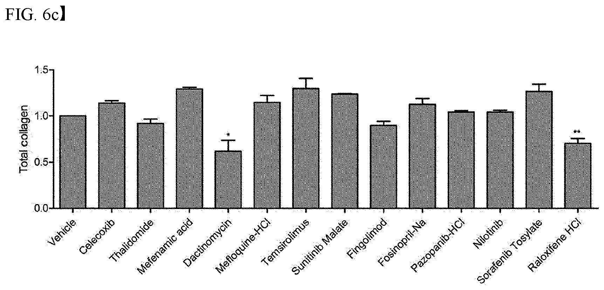

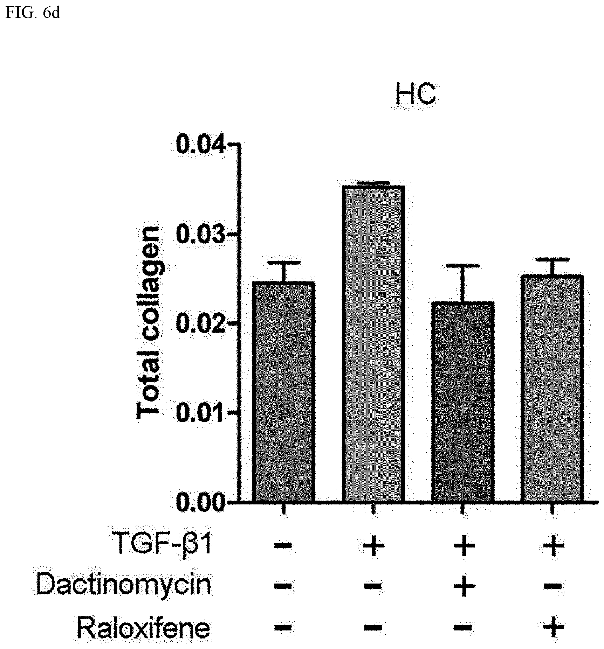

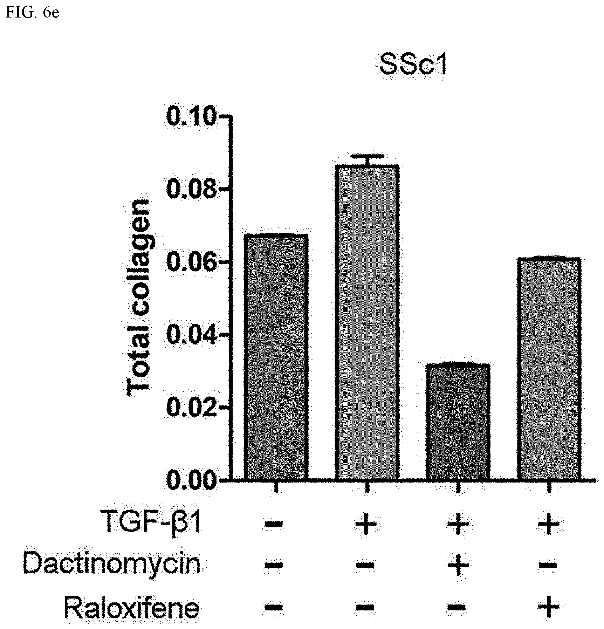

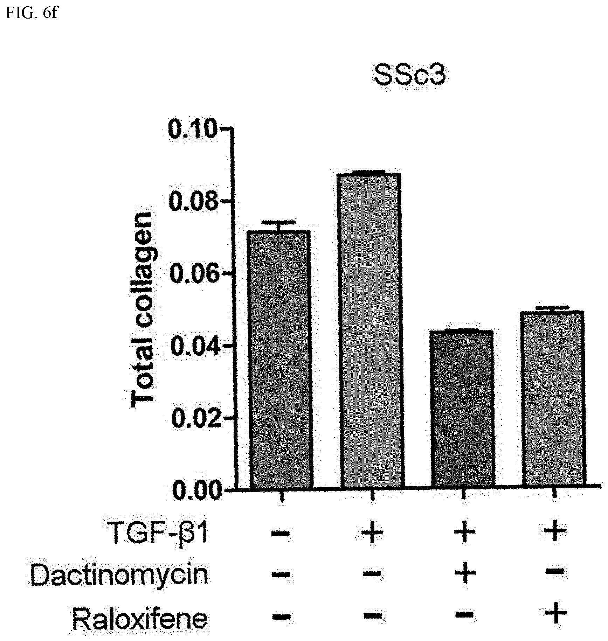

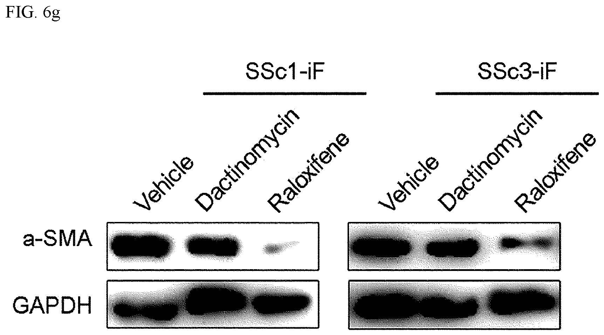

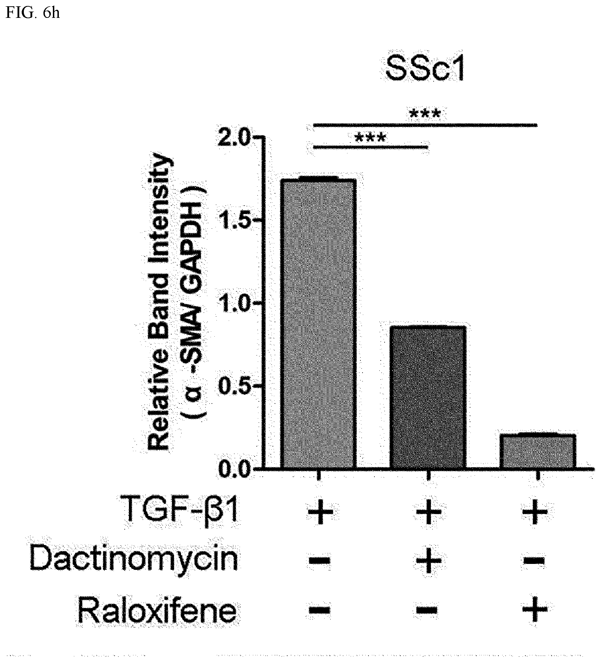

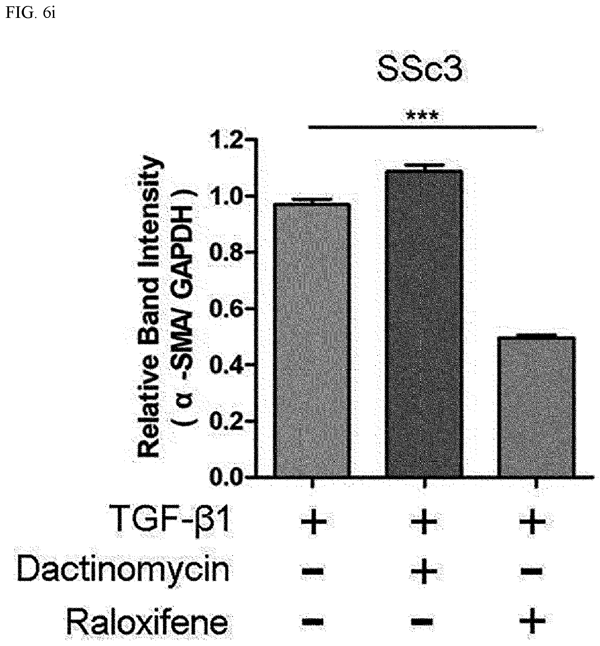

In a specific exemplary embodiment of the present invention, 800 FDA-approved drugs were treated with induced pluripotent stem cell-derived fibroblasts to primarily screen for drugs that reduce cell proliferation, and by using the primarily selected drugs, it was additionally confirmed that the expression of total collagen and the expression α-SMA were reduced, and the possibility of using the existing approved drugs as therapeutic agents for systemic sclerosis was evaluated (FIG. 6).

Through this, it was found that dactinomycin and raloxifene, which inhibit the expression of α-SMA, inhibit collagen accumulation very excellently, and thus have a prophylactic or therapeutic effect on fibrosis disease.

### [Advantageous Effects]

The fibroblasts and keratinocytes differentiated from induced pluripotent stem cells derived from patients with systemic sclerosis according to the present invention and 3D skin organoids composed of these cells are excellent systemic sclerosis disease models, and these can be used for the development of anti-fibrotic drugs and screening of the recreation technology for new drugs.

### [Description of Drawings]

FIG. 1A is a diagram showing the overall experiment outline.
FIG. 1B is a confirmation of the morphology after producing induced pluripotent stem cells by isolating PBMC from the blood of patients with systemic sclerosis.
FIG. 1C is a result of confirming the pluripotent differentiation ability of induced pluripotent stem cells by alkaline phosphatase staining.
FIG. 1D is a result of confirming the expressions of SSEA4, OCT4, TRA-1-60, SOX2, TRA-1-81, and KLF4, which are markers for induced pluripotent stem cells, by IFA.
FIG. 1E is a result of confirming the expressions of OCT4, SOX2, Nanog, and LIN28, which are markers for induced pluripotent stem cells, at the genetic level through qRT-PCR.
FIG. 1F is a result of confirming that induced pluripotent stem cells can differentiate into endoderm, mesoderm, and ectoderm using teratoma formation.
FIG. 2 is a result of confirming the differentiation of systemic sclerosis-specific induced pluripotent stem cells into keratinocytes.
FIG. 2A shows the keratinocyte differentiation protocol.
FIG. 2B is a result of confirming the morphology of keratinocytes and KRT14 and Np63, which are keratinocyte markers, with IFA,
FIG. 2C is a result of confirming the expression of OCT4, which is a marker for induced pluripotent stem cells, and the expressions of PAX6 and SOX1, which are neuroectodermal markers, and it was confirmed that the expression of OCT4, which was expressed in induced pluripotent stem cells, was reduced after differentiation, and the expression of neuroectodermal markers that were not expressed during the differentiation of keratinocytes was reduced.
FIG. 2D is a result of confirming the expressions of Np63, KRT5, and KRT14, which are keratinocyte markers, through qRT-PCR, and it was confirmed that the expression of the markers of induced pluripotent stem cells was reduced, and the expression of keratinocyte markers was increased.
FIG. 3 is a result of confirming the differentiation of systemic sclerosis-specific induced pluripotent stem cells into fibroblasts.
FIG. 3A shows the fibroblast differentiation protocol.
FIG. 3B is a result of confirming the morphology of fibroblasts and fibronectin and vimentin, which are fibroblast markers, by IFA,
FIG. 3C is a result of confirming the expression of OCT4, which is a marker for induced pluripotent stem cells, and the expressions of COL1A1, COL1A2, COL3A1, ACTA2, and vimentin, which are fibrosis markers, and it is a result of confirming that the expression of OCT4 expressed in induced pluripotent stem cells was reduced after differentiation, and the expressions of the fibrosis markers were increased, and in this case, the expressions of all of the fibrosis markers in induced pluripotent stem cell-derived fibroblasts (iPSC-fibroblast) of patients with systemic sclerosis were increased as compared to normal people.
FIG. 4 shows a method for producing a 3D skin organoid and a humanized mouse model, (A) shows a 3D skin organoid production protocol, (B) shows a mimetic diagram of a humanized mouse model production, and (C) to (J) show a 3D skin transplant protocol.
FIG. 5 shows a result of modeling systemic sclerosis *in vitro* and *in vivo.*
FIG. 5A is a proliferation diagram of induced pluripotent stem cells, showing that there is no difference in cell proliferation between induced pluripotent stem cells from normal people and induced pluripotent stem cells from patients with systemic sclerosis.
FIG. 5B is a cell proliferation diagram of iPSC-derived fibroblasts (iPSC-F), confirming that compared to induced pluripotent stem cell-derived fibroblasts (iPSC-fibroblast) from normal people, cell proliferation of systemic sclerosis induced pluripotent stem cell-derived fibroblasts (iPSC-fibroblast) was increased.
FIG. 5C is a result of confirming the expression of α-SMA, which is a systemic sclerosis marker, by western blot, confirming that compared to induced pluripotent stem cell-derived fibroblasts (iPSC-fibroblast) from normal people, the expression of α-SMA was high in induced pluripotent stem cell-derived fibroblasts (iPSC-fibroblast) from patients with systemic sclerosis.
FIG. 5D is a result of quantifying a western blot, confirming that the expression of α-SMA in systemic sclerosis was high compared to normal people (statistically significant).
FIG. 5E shows that when the total collagen in cells was quantified, the expression of collagen in induced pluripotent stem cells-F from patients with systemic sclerosis was high compared to induced pluripotent stem cells-F from normal people.
FIG. 5F is a mimetic diagram of a 3D fibroblast layer using induced pluripotent stem cells.
FIG. 5G is a result of confirming that when the 3D fibroblast layer was formed, the thickness of the 3D fibroblast layer of patients with systemic sclerosis was increased compared to normal people.
FIG. 5H is a result of confirming that when the expression of α-SMA, which is a fibrosis marker, was confirmed through IFA, the expression of α-SMA in iPSC-F from patients with systemic sclerosis was high compared to iPSC-F of normal people.
FIG. 5I is a result of confirming that when a 3D skin organoid (iSO) was produced using keratinocytes and fibroblasts differentiated from induced pluripotent stem cells and transplanted into mice, the thickness of the skin tissue of mice transplanted with iSO of patients with systemic sclerosis was increased compared to mice transplanted with iSO of normal people, and the expressions of Collagen 3 and α-SMA were increased.
FIG. 6 shows an antifibrotic drug screening using a systemic sclerosis model derived from induced pluripotent stem cells.
FIG. 6A shows a mimetic diagram of FDA-approved drug screening,
FIG. 6B shows a result of the primary screening of drugs that reduce the activated cell proliferation of iPSC-F by measuring the cell proliferation after treating 800 FDA-approved drugs in iPSC-F.
FIG. 6C is a result of confirming a decrease in the total collagen in dactinomycin and raloxifene when the total collagen was measured using the selected drugs.
FIGS. 6D to 6F are results of confirming that the total collagen expression was reduced when the two drugs were treated after applying TGF-b stimulation to induce fibrosis to iPSC-F of normal people and iPSC-F of patients with systemic sclerosis.
FIG. 6G is a result of confirming a decrease in the expression of α-SMA after treating the two drugs in iPSC-F of patients with systemic sclerosis.
FIGS. 6H to 6I show the quantified expression of α-SMA.
FIG. 7 is an *in vitro* efficacy verification result of raloxifene using a systemic sclerosis model derived from induced pluripotent stem cells.
FIG. 7A is a result of confirming that cell proliferation was reduced and wound healing, which was increased by TGF-b, was reduced when a wound healing assay was performed after treating raloxifene in iPSC-F.
FIGS. 7B to 7C show the quantified wound lengths.
FIG. 7D is a graph showing changes in the wound lengths according to the incubation time, and it is a result of confirming that the reduction width of the wound length was reduced when raloxifene was treated.
FIG. 7E is a result of confirming that the thickness of the layer, which was increased by TGF-b, was reduced when the 3D fibroblast layer was formed and treated with raloxifene.
FIGS. 7F to 7G show the quantified thickness and area of the 3D fibroblast layer.
FIG. 7H is a confirmation that when raloxifene was treated by concentration, the expression of α-SMA was reduced by concentration using western blot.
FIG. 7I shows the quantified expression of α-SMA, when raloxifene was treated by concentration.
FIG. 7J is a confirmation that when raloxifene was treated by concentration, the expression of the total collagen was reduced by concentration.
FIG. 8 is a result of verifying the efficacy of raloxifene in the bleomycin model, which is a systemic sclerosis animal model.
FIG. 8A shows the construction of a bleomycin mouse model and the drug administration protocol.
FIG. 8B is a histological analysis result of the bleomycin mouse model, confirming that the skin thickness, which was increased by bleomycin induction, was reduced by drug treatment,
FIGS. 8C to E are confirmations that the expressions of COL1A1, COL3A1, and ACTA2 were reduced in the drug treatment group when the expression of fibrosis-related genes was confirmed using qRT-PCR.
FIG. 8F is a quantitative representation that the skin thickness, which was increased by bleomycin induction, was reduced by drug treatment.
FIG. 8G is a histological analysis result of the lung tissue in the bleomycin mouse model, observing the antifibrotic effect of the lung tissue.
FIG. 9 shows a result of verifying the screening platform by evaluating the efficacy of the existing anti-fibrotic drug in a dermal fibrosis model derived from induced pluripotent stem cells. FIG. 9A is an analysis result of collagen expression, and FIG. 9B is an analysis result of fibrosis marker expression.

### [Modes of the Invention]

Hereinafter, the present invention will be described in more detail through exemplary embodiments. These exemplary embodiments are intended to illustrate the present invention more specifically, but the scope of the present invention is not limited to these exemplary embodiments.

### Example 1

### Production and characteristic analysis of induced pluripotent stem cells (iPSCs) specific to patients with systemic sclerosis (SSc)

### <1-1> Recruitment of patients and isolation of induced pluripotent stem cells

Blood from patients with systemic sclerosis was obtained from the Department of Rheumatology at Seoul St. Mary's Hospital. Whole blood was diluted with phosphate buffered saline (PBS) and centrifuged at 850 xg for 30 minutes through a Ficoll gradient. Peripheral blood cells (PBMCs) were collected and frozen. PBMCs were thawed prior to use in the experiment and resuspended in the StemSpan culture medium (STEMCELL Technological, Vancouver, British Columbia, Canada) supplemented with CC110 cytokine cocktail (STEMCELL). Cells were maintained at 5% CO₂ and 37°C for 5 days prior to reprogramming. The study was approved by the Institutional Review Board (IRB) at Seoul St. Mary's Hospital of the Catholic University of Korea.

### <1-2> Reprogramming of induced pluripotent stem cells derived from patients with systemic sclerosis

For induced pluripotent stem cells (iPSCs), peripheral blood cells (PBMCs) were isolated from the blood of patients with systemic sclerosis, and the Sendai virus including the Yamanaka factor was treated to obtain induced pluripotent stem cells. In general, induced pluripotent stem cell reprogramming refers to a technique for inducing pluripotent stem cells by artificially overexpressing factors required for reprogramming in somatic cells. Methods for overexpressing the gene include viruses, plasmid vectors, mRNA, proteins, and the like. 3 × 10⁵ PBMCs were plated on a 24-well plate. Reprogramming was induced using the CytoTune-iPSC Sendai reprogramming kit. Virus transduction was performed with a multiplex infection of 7.5 per 3 × 10⁵ cells. After treating the virus, it was centrifuged at 1,160 xg and 37°C for 30 minutes and then cultured at 5% CO₂ and 37°C. The next day, the cells were transferred to a 24-well plate coated with vitronectin (Life Technologies), centrifuged at 1,160 xg and 37°C for 10 minutes, and then cultured at 5% CO₂ and 37°C. Essential 8 (Life Technologies) was added to the culture medium at a ratio of 1:1. Reprogrammed cells were maintained and expanded in the Essential 8 culture medium by replacing the culture medium daily.

### <1-3> Confirmation of cell morphology

Reprogrammed cells were maintained and expanded in the Essential 8 culture medium by replacing the culture medium daily. In order to obtain appropriate colonies for confirmation of cell morphology, 5 × 10³ cells were plated on a 6-well plate coated with vitronectin and maintained for 5 days to confirm the morphology of induced pluripotent stem cells using the Leica DMi8 microscope (FIG. 1B).

### <1-4> AP staining

For alkaline phosphatase staining (AP staining), induced pluripotent stem cells generated and maintained through Example 1-2 above were plated by 5 × 10³ cells on a vitronectin-coated 6-well plate and expanded for 5 days. Staining of undifferentiated induced pluripotent stem cell colonies was performed using an alkaline phosphatase detection kit (Millipore, Billerica, MA, USA). The culture medium was removed, and it was fixed with 4% formaldehyde for 1 to 2 minutes. The remaining fixative was washed with PBS containing 0.05% Tween-20. Fast Red Violet, naphthol AS-BI phosphate solution, and water were mixed at a ratio of 2:1:1 and stained for 15 minutes. After washing with PBS containing 0.05% Tween-20, PBS was added to prevent drying. Stained colonies were measured using the Leica DMi8 microscope. The experiment confirmed the pluripotency of induced pluripotent stem cells (FIG. 1C).

### <1-5> Cell immunostaining

For immunofluorescence staining, the induced pluripotent stem cells that were produced and maintained through Example 1-2 above were plated by 5 × 10³ cells on a vitronectin-coated 6-well plate, and were expanded for 5 days. After expansion, it was washed with PBS and fixed with 4% formaldehyde for 30 minutes. After removing the remaining fixative with an ammonium chloride solution, 0.1% Triton X-100 (BIOSESANG) was treated to increase cell permeability. After blocking the cells for 30 minutes at room temperature with PBS containing 2% bovine serum albumin (BSA), the primary antibody was diluted in PBA at the following dilution ratio: OCT4 (1/100; Santa Cruz, CA, USA), KLF4 (1/250; Abcam, Cambridge, UK), SOX2 (1/100; BioLegend, San Diego, CA, USA), TRA-1-60 (1/100; Millipore), TRA-1-81 (1 /100; Millipore), and SSEA4 (1/200; Millipore). The primary antibody was incubated for 2 hours at room temperature. DAPI was used as a nuclear staining agent. After staining, the cells were washed and sealed using the ProLong Antifade reagent. The stained colonies were detected by the Carl Zeiss immunofluorescence microscope.

As a result, the expressions of SSEA4, OCT4, TRA-1-60, SOX2, TRA-1-81, and KLF4, which are markers for induced pluripotent stem cell, were confirmed by IFA (FIG. 1D).

### <1-6> Performance of qRT-PCR

For qRT-PCR, the total RNA was extracted using Trizol, and cDNA was synthesized using the Revert Aid TM First Strand cDNA Synthesis kit. The qRT-PCR was performed on the synthesized cDNA using LightCycle 480 SYBR Green. The primers used are presented in [Table 1]. All of the experiments were repeated three times, and the threshold of the average cycle was used to calculate gene expression for averaging GAPDH as an internal control.

**[Table 1]**

| Primer sequences for confirming pluripotent marker genes | | | | |
|---|---|---|---|---|
| **Target gene** | **Direction** | **Base sequence (5' -> 3')** | **SEQ ID NO.** | **Size** |
| **OCT4** | **Forward** | **ACCCCTGGTGCCGTGAA** | **1** | **190** |
| | **Reverse** | **GGCTGAATACCTTCCCAAATA** | **2** | |
| **SOX2** | **Forward** | **CAGCGCATGGACAGTTAC** | **3** | **321** |
| | **Reverse** | **GGAGTGGGAGGAAGAGGT** | **4** | |
| **NANOG** | **Forward** | **AAAGGCAAACAACCCACT** | **5** | **270** |
| | **Reverse** | **GCTATTCTTCGGCCAGTT** | **6** | |
| **LIN28** | **Forward** | **GTTCGGCTTCCTGTCCAT** | **7** | **122** |
| | **Reverse** | **CTGCCTCACCCTCCTTCA** | **8** | |
| **GAPDH** | **Forward** | **ACCCACTCCTCCACCTTTGA** | **9** | **101** |
| | **Reverse** | **CTGTTGCTGTAGCCAAATTCGT** | **10** | |

As a result, gene expressions of OCT4, SOX2, NANOG, and LIN28, which are markers for induced pluripotent stem cells, were confirmed (FIG. 1E).

### <1-7> Observation of teratoma formation

For observation of teratoma formation, immunodeficient mice such as SCID mice, nude mice, and the like are generally used to induce tumors, and SCID mice were used in the corresponding experiment. 1 × 10⁶ induced pluripotent stem cells produced and maintained through Examples 1-2 above were mixed with Matrigel (BD Biosciences) at a ratio of 1:1. The cells were injected into the testis of SCID mice using an insulin syringe, and tumors generated after 8 to 12 weeks were extracted and hematoxylin and eosin staining was performed.

As a result, it was confirmed that induced pluripotent stem cells having stem cell functions capable of differentiating into endoderm, mesoderm, and ectoderm were generated (FIG. IF).

### Example 2

### Differentiation of systemic sclerosis-specific induced pluripotent stem cells into keratinocytes

### <2-1> Keratinocyte differentiation protocol

The induced pluripotent stem cells generated and maintained through Example 1 above were resuspended in the Aggrewell culture medium (STEMCELL), and an embryonic body (EB) was formed using the Hanging drop culture method. The cells were incubated at 5% CO₂ and 37°C for one day. The formed EB was harvested and maintained by adding 1 ng/mL BMP4 to the Essential 8 culture medium the next day.

The EB was harvested and attached to a collagen IV-coated plate. During the differentiation period, the culture medium was changed once every 2 days. The culture medium was replaced by Keratin Differentiation Culture Medium 1 (DMEM/F12 3:1, 2% fetal bovine serum (FBS), 0.3 mmol/L L-ascorbic acid, 5 µg/mL insulin, and 24 µg/mL adenine), and it was maintained for 7 days by adding 3 µM retinoic acid (RA), 25 ng/mL BMP4, and 20 ng/mL EGF. After 7 days (Day 8), the culture medium was replaced by Keratin Differentiation Culture Medium 2 (defined keratinocyte serum-free medium, 0.3 mmol/L L-ascorbic acid, 5 µg/mL insulin, and 10 µg/mL adenine), and 3 µM retinoic acid (RA), 25 ng/mL BMP4, and 20 ng/mL EGF were added and maintained for 4 days. After 4 days (Day 12), the culture medium was replaced by Keratin Differentiation Culture Medium 3 (defined keratinocyte serum-free medium and keratinocyte serum-free medium (1:1)), and 10 ng/mL BMP4 and 20 ng/mL EGF were added to maintain and expand differentiated keratinocytes (FIG. 2A).

### <2-2> Confirmation of morphology of keratinocytes and markers

The keratinocytes formed in Example 2-1 above were harvested on Day 21 after the differentiation started to confirm the morphology using the Leica DMi8 microscope. The harvested cells were washed with PBS, and the cells were fixed with 4% formaldehyde for 30 minutes for immunofluorescence staining. Afterwards, the remaining fixative was removed with an ammonium chloride solution, and then it was treated with 0.1% Triton X-100 (BIOSESANG) to increase cell permeability. After blocking the cells for 30 minutes at room temperature with PBS containing 2% bovine serum albumin (BSA), the primary antibody was diluted in PBA at the following dilution ratio: p63 (1/100; Abcam, Cambridge, UK) and KRT14 (1/100; Abcam, Cambridge, UK). The primary antibody was incubated for 2 hours at room temperature. DAPI was used as a nuclear staining agent. After staining, the cells were washed and sealed using the ProLong Antifade reagent. The stained keratinocytes were detected by the Carl Zeiss immunofluorescence microscope.

As a result, the expressions of p63 and KRT14, which are markers of keratinocytes, were confirmed by IFA (FIG. 2B).

### <2-3> Confirmation of decreased expression of induced pluripotent stem cells and neuroectodermal markers

The keratinocytes formed in Example 2-1 above were harvested on Day 21 after the differentiation started, and for qRT-PCR, the total RNA was extracted using Trizol, and cDNA was synthesized using the Revert Aid TM First Strand cDNA Synthesis kit. The qRT-PCR was performed on the synthesized cDNA using LightCycle 480 SYBR Green. The primers used are presented in [Table 2]. All of the experiments were repeated three times, and the threshold of the average cycle was used to calculate gene expression for averaging GAPDH as an internal control.

As a result, it was confirmed that the expression of OCT4, which is an induced pluripotent stem cell marker, and the expressions of PAX6 and SOX1, which are neuroectoderm markers, were reduced (FIG. 2C).

### <2-4> Confirmation of expression of keratinocyte markers using qRT-PCR

The keratinocytes formed in Example 2-1 above were harvested on Day 21 after the differentiation started, and for qRT-PCR, the total RNA was extracted using Trizol, and cDNA was synthesized using the Revert Aid TM First Strand cDNA Synthesis kit. The qRT-PCR was performed on the synthesized cDNA using LightCycle 480 SYBR Green. The primers used are presented in [Table 2]. All of the experiments were repeated three times, and the threshold of the average cycle was used to calculate gene expression for averaging GAPDH as an internal control.

**Table 2**

| Primer sequences for confirming keratinocyte differentiation markers | | | | |
|---|---|---|---|---|
| **Target gene** | **Direction** | **Base sequence (5' -> 3')** | **SEQ ID NO.** | **Size** |
| **hOCT4** | **Forward** | **ACCCCTGGTGCCGTGAA** | **11** | **190** |
| | **Reverse** | **GGCTGAATACCTTCCCAAATA** | **12** | |
| **hPAX6** | **Forward** | **GTCCATCTTTGCTTGGGAAA** | **13** | **110** |
| | **Reverse** | **TAGCCAGGTTGCGAAGAACT** | **14** | |
| **hSOX1** | **Forward** | **CACAACTCGGAGATCAGCAA** | **15** | **133** |
| | **Reverse** | **GGTACTTGTAATCCGGGTGC** | **16** | |
| **hNp63** | **Forward** | **GGAAAACAATGCCCAGACTC** | **17** | **294** |
| | **Reverse** | **GTGGAATACGTCCAGGTGGC** | **18** | |
| **hKRT5** | **Forward** | **ACCGTTCCTGGGTAACAGAGCCAC** | **19** | **198** |
| | **Reverse** | **GCGGGAGACAGACGGGGTGATG** | **20** | |
| **hKRT14** | **Forward** | **GCAGTCATCCAGAGATGTGACC** | **21** | **181** |
| | **Reverse** | **GGGATCTTCCAGTGGGATCT** | **22** | |
| **hGAPDH** | **Forward** | **ACCCACTCCTCCACCTTTGA** | **23** | **110** |
| | **Reverse** | **CTGTTGCTGTAGCCAAATTCGT** | **24** | |

As a result, it was confirmed that the expressions of Np63, KRT5, and KRT14, which are markers of keratinocytes, were increased (FIG. 2D).

### Example 3

### Differentiation of systemic sclerosis-specific induced pluripotent stem cells into fibroblasts

### <3-1> Fibroblast differentiation protocol

The induced pluripotent stem cells generated and maintained through Example 1 above were resuspended in the Aggrewell culture medium (STEMCELL), and an embryonic body (EB) was formed using the Hanging drop culture method. The cells were cultured at 5% CO₂ and 37°C for one day. The formed EB was harvested and maintained in the Essential 8 culture medium. The next day, the EB was harvested and attached to a Matrigel-coated plate. The culture medium was replaced with Fibroblast Differentiation Culture Medium 1 (DMEM/F12 3:1, 5% fetal bovine serum (FBS), 5 µg/mL insulin, 0.18 mM adenine, and 10 ng/mL epidermal growth factor (EGF)). During the differentiation period, the culture medium was changed once every 2 days. After 3 days (Day 4), 0.5 nM BMP4 was added to Fibroblast Differentiation Culture Medium 1 and maintained for 3 days. Afterwards, the culture medium was replaced with Fibroblast Differentiation Culture Medium 2 (DMEM/F12 1:1, 5% FBS, 1% nonessential amino acids) and maintained for 7 days. After seven days (Day 14), the expanded cells were transferred to an uncoated plate. In this case, the culture medium was replaced with Fibroblast Differentiation Culture Medium 1. After seven days (Day 21), the expanded cells were transferred to a collagen 1-coated plate. The culture medium was replaced with Fibroblast Differentiation Culture Medium 1 once every 2 days to maintain and expand the differentiated fibroblasts (FIG. 3A).

### <3-2> Confirmation of the morphology of fibroblasts and markers

The fibroblasts formed in Example 3-1 above were harvested on Day 28 after the differentiation started, and the morphology was confirmed using the Leica DMi8 microscope. The harvested cells were washed with PBS, and the cells were fixed with 4% formaldehyde for 30 minutes for immunofluorescence staining. Afterwards, the remaining fixative was removed with an ammonium chloride solution, and then, 0.1% Triton X-100 (BIOSESANG) was treated to increase cell permeability. After blocking the cells for 30 minutes at room temperature with PBS containing 2% bovine serum albumin (BSA), the primary antibody was diluted in PBA at the following dilution ratio: Fibronectin (1/200; Abcam, Cambridge, UK) and Vimentin (1/200; Abcam, Cambridge, UK). The primary antibody was incubated for 2 hours at room temperature. DAPI was used as a nuclear staining agent. After staining, the cells were washed and sealed using the ProLong Antifade reagent. The stained keratinocytes were detected by the Carl Zeiss immunofluorescence microscope.

As a result, the expressions of fibronectin and vimentin, which are cell markers of fibroblasts, were confirmed by IFA (FIG. 3B).

### <3-3> Confirmation of expression of induced pluripotent stem cells and fibrosis markers

The fibroblasts formed in Example 3-1 above were harvested on Day 28 after the differentiation started. For qRT-PCR, the total RNA was extracted using Trizol, and cDNA was synthesized using the Revert Aid TM First Strand cDNA Synthesis kit. The qRT-PCR was performed on the synthesized cDNA using LightCycle 480 SYBR Green. The primers used are presented in [Table 3]. All of the experiments were repeated three times, and the threshold of the average cycle was used to calculate gene expression for averaging GAPDH as an internal control.

**[Table 3]**

| **Primer sequences for confirmation of expression of fibroblast differentiation markers and fibrosis factors** | | | | |
|---|---|---|---|---|
| **Target gene** | **Direction** | **Base sequence (5' -> 3')** | **SEQ ID NO.** | **Size** |
| **hOCT4** | **Forward** | **ACCCCTGGTGCCGTGAA** | **11** | **190** |
| | **Reverse** | **GGCTGAATACCTTCCCAAATA** | **12** | |
| **hCOL1A1** | **Forward** | **CCCCTGGAAAGAATGGAGATG** | **25** | **148** |
| | **Reverse** | **TCCAAACCACTGAAACCTCTG** | **26** | |
| **hCOL1A2** | **Forward** | **GGATGAGGAGACTGGCAACC** | **27** | **77** |
| | **Reverse** | **TGCCCTCAGCAACAAGTTCA** | **28** | |
| **hCOL3A1** | **Forward** | **CGCCCTCCTAATGGTCAAGG** | **29** | **161** |
| | **Reverse** | **TTCTGAGGACCAGTAGGGCA** | **30** | |
| **hACTA2** | **Forward** | **AAAGCAAGTCCTCCAGCGTT** | **31** | **115** |
| | **Reverse** | **TTCACAGGATTCTGGGAGCG** | **32** | |
| **hGAPDH** | **Forward** | **ACCCACTCCTCCACCTTTGA** | **33** | **110** |
| | **Reverse** | **CTGTTGCTGTAGCCAAATTCGT** | **34** | |

As a result, it was confirmed that the expression of OCT4, which was expressed in induced pluripotent stem cells, was reduced after differentiation, and the expressions of the fibroblast markers were increased. In this case, it was confirmed that the expressions of the fibrosis markers were increased in patients with systemic sclerosis compared to normal people (FIG. 3C).

Through the above results, the same trend as the clinical symptoms of systemic sclerosis was confirmed, and disease modeling *in vitro* of fibroblasts differentiated from induced pluripotent stem cells in patients with systemic sclerosis was verified.

### [Example 4]

### Construction of 3D skin organoids and humanized mouse models

### <4-1> 3D skin organoid production protocol

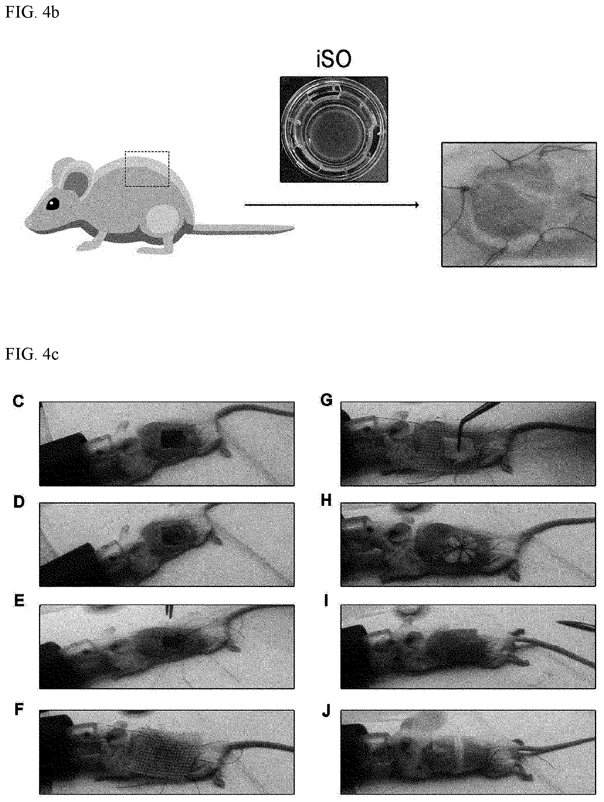

3D skin organoids were produced using keratinocytes and fibroblasts formed in Examples 2 and 3 above. 2 × 10⁵ fibroblasts after 28 days of differentiation were harvested and mixed with collagen 1 and plated on a 6-well-sized Transwell plate. It was replaced once every 2 days with Fibroblast Differentiation Culture Medium 1 for 5 days. When collagen 1 was gelated, 1 × 10⁶ keratinocytes after 21 days of differentiation were harvested, and it was resuspended in 50 µL to 100 µL of Epithelial Cell Culture Medium 1 (Epithelial Medium; EP1; DMEM/F12 3:1, 4 mM L-glutamine, 40 µM adenine, 10 µg/mL transferrin, 10 µg/mL insulin, and 0.1% FBS) and spread on fibroblasts. After maintaining for 2 days (Day 7), the culture medium was replaced with Epithelial Cell Culture Medium 2 (Epithelial Medium; EP2; prepared by adding 1.8 mM calcium chloride to the EP1 culture medium) and cultured for 2 days. After 2 days (Day 9), it was replaced with Epithelial Cell Culture Medium 3 (Epithelial Medium; EP3; DMEM/F12 1:1, 4 mM L-glutamine, 40 µM adenine, 10 µg/mL transferrin, 10 µg/mL insulin, 2% FBS, and 1.8 mM calcium chloride). In this case, the culture medium was added only to the bottom of the insert of the transwell plate and maintained by the air-liquid interface culture method (FIG 4A).

### <4-2> Mimetic diagram of production of humanized mouse model and 3D skin transplantation protocol

3D skin organoids formed through Example 4-1 above were transplanted into mice using a suture-fixing dressing method (FIG. 4B). The skin tissue of the anesthetized SCID mice was cut to 1 × 2 cm (FIG. 4C), and 3D skin organoids derived from induced pluripotent stem cells were raised (FIG. 4D). The mouse skin and skin organoid to be transplanted were sutured 8 to 9 times (FIG. 4E). After placing a gauze on the suture, it was tied with the remaining suture thread after suturing (FIGS. 4F to H). After dressing with a band, it was maintained while watching the progress of the mice (FIGS. 4I to J).

### Example 5

### Identification of characteristics of systemic sclerosis disease modeling in vitro and in vivo

### <5-1> Analysis of cell proliferation in vitro

5 × 10³ cells were plated on a 96-well plate for analysis of proliferation of induced pluripotent stem cells derived from patients with systemic sclerosis and fibroblasts derived from the induced pluripotent stem cells formed in Examples 1 and 3 above. For cell proliferation, absorbance was measured at 450 nm by using the Cell Counting Kit-8 (Dojindo).

As a result, there was no difference in cell proliferation between the induced pluripotent stem cells from normal people and the induced pluripotent stem cells from patients with systemic sclerosis (FIG. 5A), but it was confirmed that the cell proliferation of the fibroblasts derived from the induced pluripotent stem cells from patients with systemic sclerosis was increased, compared to the fibroblasts derived from induced pluripotent stem cells derived from normal people (FIG. 5B).

Through the above results, the same trend as the clinical symptoms of systemic sclerosis was confirmed, and disease modeling *in vitro* of fibroblasts differentiated from induced pluripotent stem cells in patients with systemic sclerosis was verified.

### <5-2> Analysis of expression of fibrosis factors in vitro

In order to confirm the expressions of fibrosis factors of the fibroblasts derived from the induced pluripotent stem cells of patients with systemic sclerosis formed in Example 3 above, 1 × 10⁶ fibroblasts were plated on a 100 mm culture dish and maintained and expanded for 5 days. The protein was extracted using the RIPA buffer (Sigma) to analyze the expression of fibrosis factors, and the protein concentration was quantified by the Bradford assay. The same concentration of the protein was loaded on 10% SDS-PAGE, and the protein was transferred to polyvinylidene difluoride membranes. After blocking for 1 hour at room temperature with PBS containing 5% skim milk, the primary antibody was diluted in PBS containing 5% skim milk at the following dilution ratio: α-SMA (1/200; Abcam). The primary antibody was incubated for 2 hours at room temperature. After washing with PBS containing 0.1% Tween-20, the peroxidase-linked IgG secondary antibody was incubated, and protein expression was measured using the ECL kit.

As a result, it was confirmed that the expression of α-SMA, which is a fibrosis factor, was increased in the fibroblasts derived from the induced pluripotent stem cells of patients with systemic sclerosis, compared to the fibroblasts derived from the induced pluripotent stem cells of normal people (FIG. 5C). When it was quantified using the image J program, a statistically significant increase was confirmed (FIG. 5D).

Through the above results, the same trend as the clinical symptoms of systemic sclerosis was confirmed, and disease modeling *in vitro* of fibroblasts differentiated from induced pluripotent stem cells of patients with systemic sclerosis was verified.

### <5-3> Analysis of collagen expression in vitro

In order to confirm collagen expression of the fibroblasts derived from the induced pluripotent stem cells of patients with systemic sclerosis formed in Example 3 above, 1 × 10⁶ fibroblasts were plated on a 100 mm culture dish and maintained and expanded for 5 days. For collagen expression, the hydroxyproline assay kit (Sigma) was used. The cells or cell culture media were harvested, mixed with hydrochloric acid at a ratio of 1:1, and cultured at 120°C for 3 hours. After culture, 5 mg of activated charcoal was added, followed by centrifugation at 13,000 xg for 5 minutes to separate only the supernatant. The harvested supernatant was transferred to a 96-well plate and dried completely at 60°C. The Chloramine T/Oxidation Buffer mixture was added to the well, and it was cultured for 5 minutes at room temperature. After adding the diluted DMAB Reagent and incubating at 60°C for 90 minutes, absorbance at 560 nm was measured.

As a result, it was confirmed that the expression of the total collagen, which is a fibrosis factor, was increased in the fibroblasts derived from induced pluripotent stem cells of patients with systemic sclerosis, compared to the fibroblasts derived from the induced pluripotent stem cells of normal people (FIG. 5E).

Through the above results, the same trend as the clinical symptoms of systemic sclerosis was confirmed, and disease modeling *in vitro* of fibroblasts differentiated from induced pluripotent stem cells in patients with systemic sclerosis was verified.

### <5-4> Analysis of 3D fibroblast layer in vitro

A 3D fibroblast layer was produced using the fibroblasts formed in Example 3 above. 2 × 10⁵ fibroblasts after 28 days of differentiation were harvested and mixed with collagen 1 and plated on a 6-well-sized Transwell plate. It was replaced once every 2 days with Fibroblast Differentiation Culture Medium 1 for 5 days (FIG. 5F).

As a result, it was confirmed that the thickness of the 3D fibroblast layer produced using the fibroblasts derived from the induced pluripotent stem cells of patients with systemic sclerosis was increased, compared to the fibroblasts derived from the induced pluripotent stem cells of normal people (FIG. 5G).

Through the above results, the same trend as the clinical symptoms of systemic sclerosis was confirmed, and disease modeling *in vitro* of fibroblasts differentiated from induced pluripotent stem cells of patients with systemic sclerosis was verified.

### <5-5> Analysis of expression of fibrosis factors in vitro

The fibroblasts formed in Example 3-1 above were harvested on Day 28 after the differentiation started and washed with PBS, and the cells were fixed for 30 minutes with 4% formaldehyde for immunofluorescence staining. Afterwards, the remaining fixative was removed with an ammonium chloride solution, and then 0.1% Triton X-100 (BIOSESANG) was treated to increase cell permeability. After blocking the cells for 30 minutes at room temperature with PBS containing 2% bovine serum albumin (BSA), the primary antibody was diluted in PBA at the following dilution ratio: α-SMA (1/200; Abcam). The primary antibody was incubated for 2 hours at room temperature. DAPI was used as a nuclear staining agent. After staining, the cells were washed and sealed using the ProLong Antifade reagent. The expression of α-SMA, which is a fibrosis factor of fibroblasts, was confirmed by IFA using the Carl Zeiss immunofluorescence microscope.

As a result, it was confirmed that the expression of α-SMA, which is a fibrosis factor, was increased in the fibroblasts derived from the induced pluripotent stem cells of patients with systemic sclerosis, compared to the fibroblasts derived from the induced pluripotent stem cells of normal people (FIG. 5H).

Through the above results, the same trend as the clinical symptoms of systemic sclerosis was confirmed, and disease modeling *in vitro* of fibroblasts differentiated from induced pluripotent stem cells in patients with systemic sclerosis was verified.

### <5-6> Production of systemic sclerosis modeling in vivo and histological analysis

The 3D skin organoids derived from the induced pluripotent stem cells of normal people and patients with systemic sclerosis formed in Example 4-1 above were transplanted into SCID mice. After 2 weeks after transplantation, the skin tissue was harvested, and paraffin blocks were prepared for staining analysis. The harvested skin tissue was fixed in 4% formaldehyde. The tissue was maintained in the running water for one day to remove the remaining fixative. Dehydration was carried out sequentially while increasing the concentration of an ethanol solution. After dehydration, a transparent process was performed with xylene, followed by paraffin penetration. The next day, the tissue was fixed to a paraffin block, and a tissue section was obtained using a microtome. The slide was dried at 60°C for 1 hour. After the slide was deparaffinized with xylene, it was rehydrated while reducing the concentration of the ethanol solution and washed with tap water, and staining was performed

For hematoxylin and eosin (H&E) staining, sections were incubated in the Harris hematoxylin solution for 10 minutes. The slide was washed, decolorized in an ethanol solution containing 1% HCl, and then neutralized in 0.2% ammonia water. Control staining was performed with an eosin solution for 1 minute and 30 seconds. The slide was washed and subjected to a dehydration process while increasing the concentration of ethanol. After removing the remaining ethanol with xylene, it was sealed using the VectaMount Permanent Mounting Medium.

For Masson Trichrome staining, the slide was mordated overnight in the Bouin solution. After washing the slide, nuclei were stained for 10 minutes with Weigert Iron Hematoxylin. The cytoplasm was stained for 5 minutes with Biebrich scarlet-acid fuchsin. It was decolorized for 10 minutes with a solution in which phosphotungstic acid, phosphomolybdic acid, and DW were mixed at a ratio of 1:1:2. The collagenic fiber was stained by maintaining in a 2% aniline blue solution for 5 minutes. The slide was washed and subjected to a dehydration process while increasing the concentration of ethanol. The remaining ethanol was removed with xylene, and it was sealed using the VectaMount Permanent Mounting Medium.

The slide was maintained for 1 hour in a Picrosirius Red solution for Picrosirius Red staining. It was washed with acetic acid and subjected to a dehydration process while increasing the concentration of ethanol. After removing the remaining ethanol with xylene, it was sealed using the VectaMount Permanent Mounting Medium.

For immunochemical staining, it was cultured for 15 minutes in 3% hydrogen peroxide to block endogenous peroxidase. After washing the slide, the cells were blocked for 1 hour at room temperature with PBS containing 1% bovine serum albumin (BSA), and then, the primary antibody was diluted in PBA at the following dilution ratio: collagen 3 (1/200; Abcam) and α-SMA (1/200; Abcam). The primary antibody was incubated at room temperature at 4°C for one day. The next day, it was washed with Tris buffered saline (TBS) containing 0.1% Tween-20 (TBST), and after the secondary antibody was maintained at room temperature for 10 minutes, it was cultured in the ABC reagent for 10 minutes. The slide was washed with PBS, and the DAB solution was maintained for 1 minute. Mayer's hematoxylin was applied for 1 minute as a nuclear staining agent. The slide was washed and subjected to a dehydration process while increasing the concentration of ethanol. The remaining ethanol was removed with xylene, and it was sealed using the VectaMount Permanent Mounting Medium.

As a result, it was confirmed that the skin thickness of the mice transplanted with the 3D skin organoid derived from the induced pluripotent stem cells of patients with systemic sclerosis was increased, compared to the mice transplanted with the 3D skin organoid derived from the induced pluripotent stem cells of normal people. In addition, it was confirmed that the expressions of collagen 3 and α-SMA were increased in the mice transplanted with the 3D skin organoid derived from the induced pluripotent stem cells of patients with systemic sclerosis (FIG 5I).

Through the above results, the same trend as the clinical symptoms of systemic sclerosis was confirmed, and disease modeling *in vivo* of cells differentiated from induced pluripotent stem cells of patients with systemic sclerosis was verified.

### Example 6

### Antifibrotic drug screening using systemic sclerosis model derived from induced pluripotent stem cells

### <6-1> FDA-approved drug screening

In order to analyze the proliferation of the fibroblasts derived from the induced pluripotent stem cells formed in Example 3 above, 5 × 10³ cells were plated on a 96-well plate. The plated cells were treated with about 800 FDA-approved drugs to screen for drugs that reduce the proliferation (FIG. 6A). For cell proliferation, absorbance was measured at 450 nm by using the Cell Counting Kit-8 (Dojindo) (FIG. 6B).

In order to detect drugs that reduce the total collagen expression using the selected drug, 1 × 10⁶ fibroblasts were plated on a 100 mm culture dish as in Example 5-3 above, treated with the drug, and then maintained and expanded. For collagen expression, the hydroxyproline assay kit (Sigma) was used. The cells or cell culture media were harvested, mixed with hydrochloric acid at a ratio of 1:1, and incubated at 120°C for 3 hours. After incubation, 5 mg of activated charcoal was added, followed by centrifugation at 13,000 xg for 5 minutes to separate only the supernatant. The harvested supernatant was transferred to a 96-well plate and dried completely at 60°C. The Chloramine T/Oxidation Buffer mixture was added to the well, and it was incubated for 5 minutes at room temperature. After adding the diluted DMAB Reagent and incubating at 60°C for 90 minutes, absorbance at 560 nm was measured.

As a result, it was confirmed that dactinomycin and raloxifene decreased the cell proliferation and increased the total collagen expression (FIGS. 6C to F).

### <6-2> Analysis of expression of fibrosis factors in vitro of target drug

In order to verify the antifibrotic effect of dactinomycin and raloxifene, which are the drugs selected through the above experiment, 1 × 10⁶ fibroblasts were plated on a 100 mm culture dish, treated with the target drugs, and maintained and expanded. The protein was extracted using the RIPA buffer (Sigma) to analyze the expression of fibrosis factors, and the protein concentration was quantified by the Bradford assay. The same concentration of the protein was loaded on 10% SDS-PAGE, and the protein was transferred to polyvinylidene difluoride membranes. After blocking for 1 hour at room temperature with PBS containing 5% skim milk, the primary antibody was diluted in PBS containing 5% skim milk at the following dilution ratio: α-SMA (1/200; Abcam). The primary antibody was incubated for 2 hours at room temperature. After washing with PBS containing 0.1% Tween-20, the peroxidase-linked IgG secondary antibody was incubated, and protein expression was measured using the ECL kit.

As a result, it was confirmed that raloxifene effectively decreased the expression of α-SMA compared to dactinomycin (FIGS. 6G to I).

### Example 7

### Verification of anti-fibrotic effect of raloxifene in vitro

### <7-1> Confirmation of reduction in cell proliferation in vitro by treatment with raloxifene

A wound healing experiment was conducted to confirm the inhibitory effect of cell proliferation of raloxifene, which is the drug selected in Example 6 above. The fibroblasts were plated on a 6-well plate and maintained and expanded. When the cells filled the plate, the plate was scratched, and fibrosis was induced by TGF-b and it was treated with the drug raloxifene.

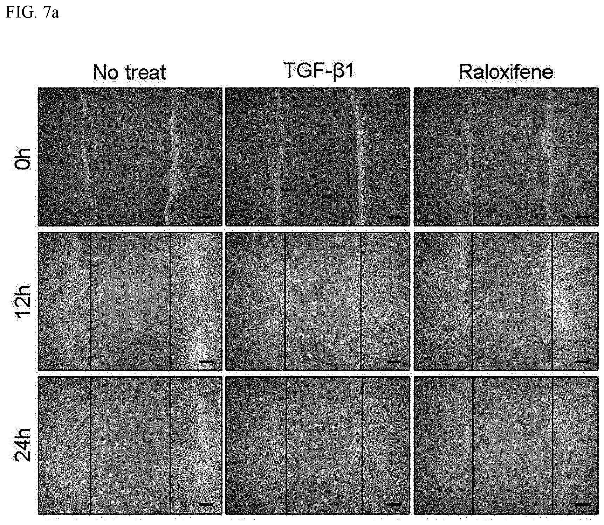

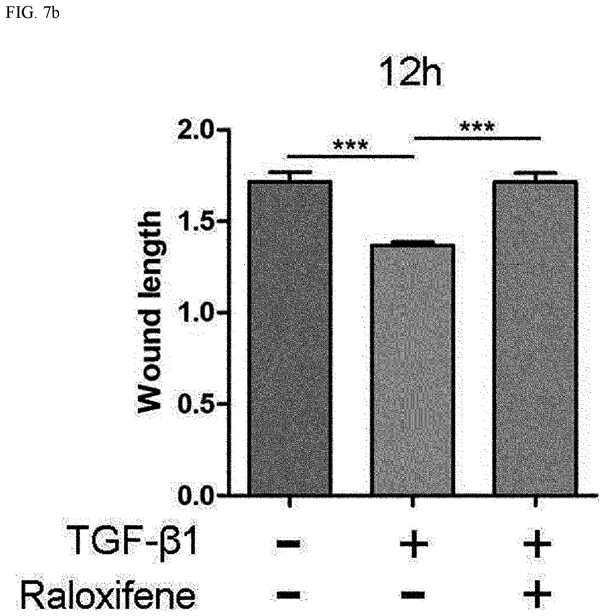

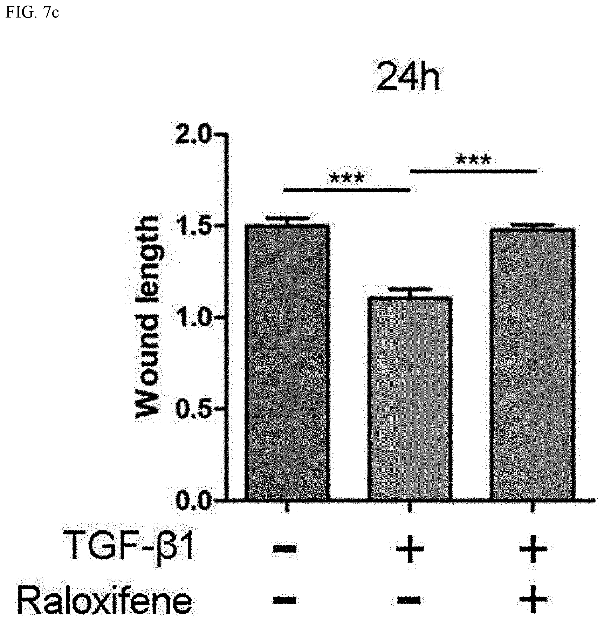

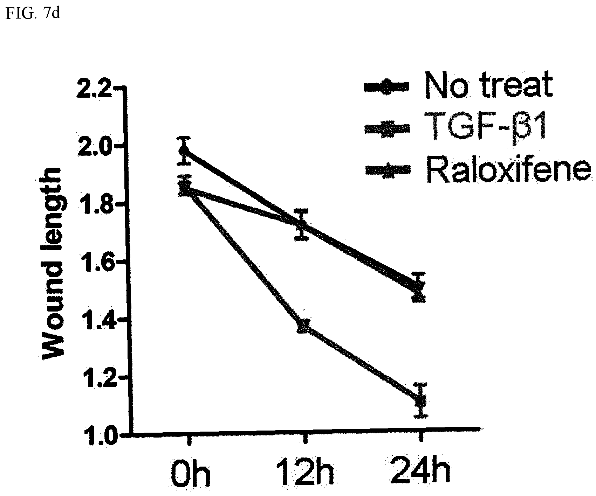

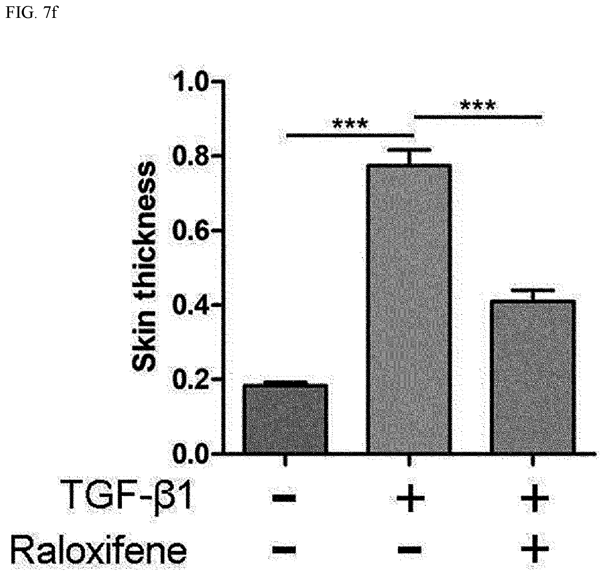

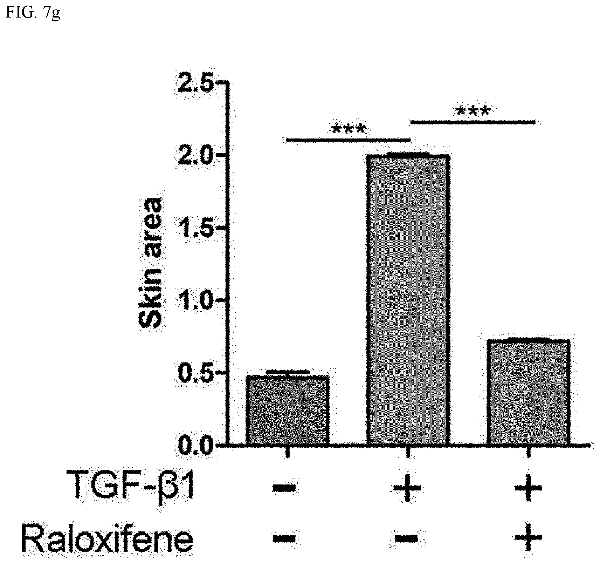

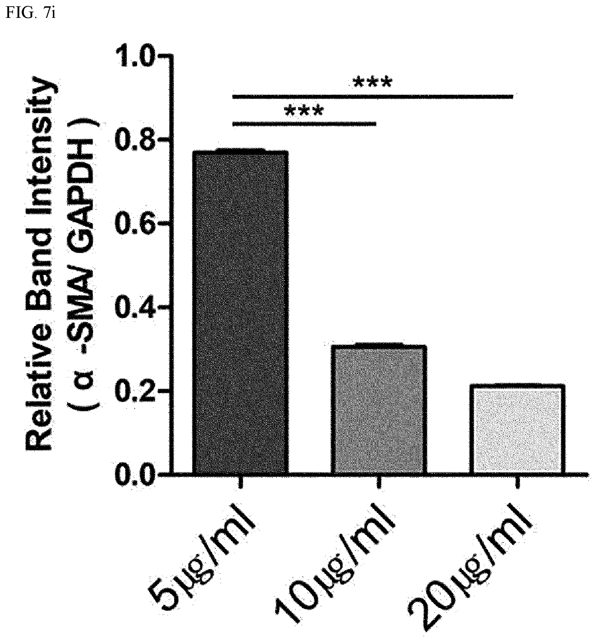

As a result, it was confirmed that the proliferation of the cells, which was increased by TGF-b, was reduced by raloxifene (FIGS. 7A to D). Through the above results, it was verified that raloxifene is effective in reducing cell proliferation.

### <7-2> Confirmation of reduction in thickness of 3D fibroblast layer by raloxifene treatment

In order to confirm the effect of reducing the skin thickness by raloxifene, which is the drug selected in Example 6 above, fibrosis was induced in the 3D fibroblast layer with TGF-b, and it was treated with the drug raloxifene.

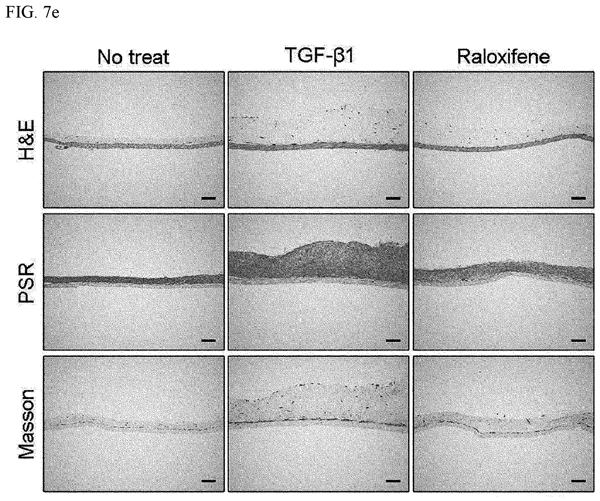

As a result, it was confirmed that the thickness of the 3D fibroblast layer, which was increased by TGF-b, was reduced by raloxifene (FIGS. 7E to G). Through the above results, it was verified that raloxifene is effective in reducing the thickness of the 3D fibroblast layer.

### <7-3> Confirmation of reduction of expression of fibrosis factors by raloxifene treatment

In order to confirm the reduction effect of the expression of the fibrosis factors of raloxifene, which is the drug selected in Example 6, raloxifene was treated by concentration, and the expression of α-SMA was confirmed. 1 × 10⁶ fibroblasts were plated on a 100 mm culture dish, treated with raloxifene, and maintained and expanded. The protein was extracted using the RIPA buffer (Sigma) to analyze the expression of fibrosis factors, and the protein concentration was quantified by the Bradford assay. The same concentration of the protein was loaded on 10% SDS-PAGE, and the protein was transferred to polyvinylidene difluoride membranes. After blocking for 1 hour at room temperature with PBS containing 5% skim milk, the primary antibody was diluted in PBS containing 5% skim milk at the following dilution ratio: α-SMA (1/200; Abcam). The primary antibody was incubated for 2 hours at room temperature. After washing with PBS containing 0.1% Tween-20, the peroxidase-linked IgG secondary antibody was incubated, and protein expression was measured using the ECL kit.

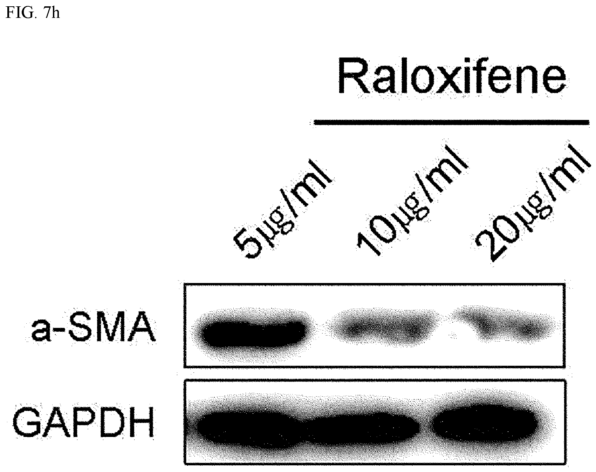

As a result, it was confirmed that the expression of α-SMA was reduced as the treatment concentration of raloxifene was increased (FIGS. 7H to I).

Next, raloxifene was treated by concentration and changes in the total collagen expression were confirmed. In the same manner as in Example 5-3 above, 1 × 10⁶ fibroblasts were plated on a 100 mm culture dish, treated with the drug, and maintained and expanded. For collagen expression, the hydroxyproline assay kit (Sigma) was used. The cells or cell culture media were harvested, mixed with hydrochloric acid at a ratio of 1:1, and incubated at 120°C for 3 hours. After incubation, 5 mg of activated charcoal was added, followed by centrifugation at 13,000 xg for 5 minutes to separate only the supernatant. The harvested supernatant was transferred to a 96-well plate and dried completely at 60°C. The Chloramine T/Oxidation Buffer mixture was added to the well, and it was incubated for 5 minutes at room temperature. After adding the diluted DMAB Reagent and incubating at 60°C for 90 minutes, absorbance at 560 nm was measured.

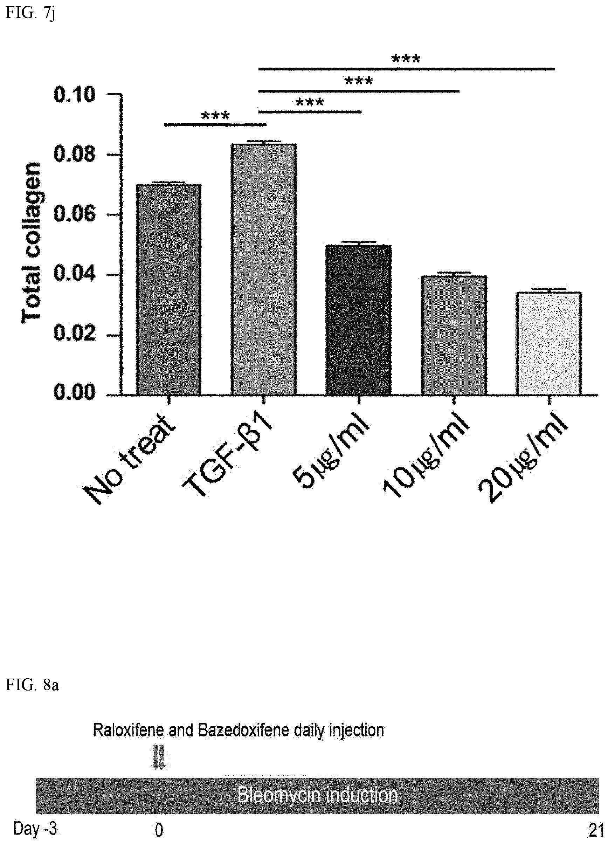

As a result, it was confirmed that the expression of the total collagen was reduced as the treatment concentration of raloxifene was increased (FIG. 7J).

### Example 8

### Verification of efficacy of raloxifene in bleomycin model, a systemic sclerosis animal model

### <8-1> Bleomycin model establishment protocol

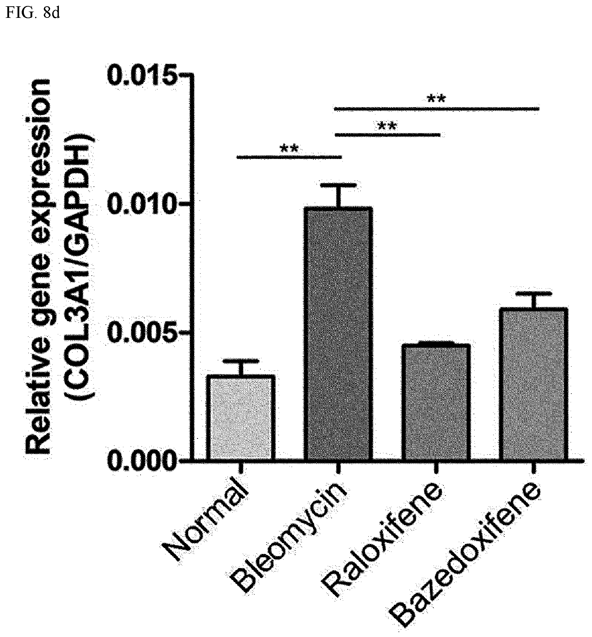

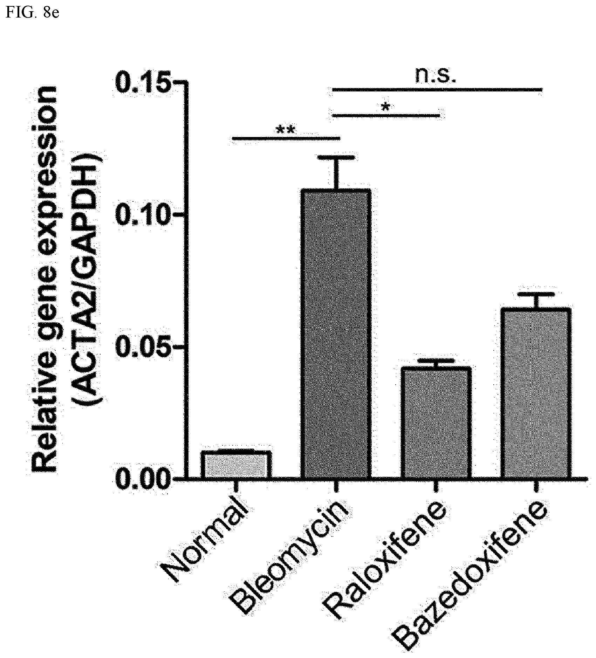

A systemic sclerosis model was established by dissolving Bleocin (Dong-A ST, 15 mg) in PBS and injecting it daily into the nape of mice. Raloxifene and bazedoxifene were injected subcutaneously for 21 days from 3 days after the injection of Bleocin (FIG. 8A).

### <8-2> Analysis of expression of fibrosis factors by treatment with raloxifene in bleomycin model

ECM gene expression was confirmed to verify the antifibrotic efficacy of raloxifene in the bleomycin model established in Example 8-1 above. After harvesting the skin tissue, the total RNA was extracted using Trizol, and cDNA was synthesized using the Revert Aid TM First Strand cDNA Synthesis kit. The qRT-PCR was performed on the synthesized cDNA using LightCycle 480 SYBR Green. The primers used are presented in [Table 4]. All of the experiments were repeated three times, and the threshold of the average cycle was used to calculate gene expression for averaging GAPDH as an internal control.

**Table 4**

| Primer sequences for confirming expression of fibrosis factors | | | | |
|---|---|---|---|---|
| **Target gene** | **Direction** | **Base sequence (5' -> 3')** | **SEQ ID NO.** | **Size** |
| **mCOL1A1** | **Forward** | **GCAACAGTCGCTTCACCTACA** | **35** | **138** |
| | **Reverse** | **CAATGTCCAAGGGAGCCACAT** | **36** | |
| **mCOL3A1** | **Forward** | **TGAGCGTGGCTATTCCTTCGT** | **37** | **76** |
| | **Reverse** | **GCCGTGGCCATCTCATTTTCAA** | **38** | |
| **mACTA2** | **Forward** | **GTTCTAGAGGATGGCTGTACTA** | **39** | **108** |
| | **Reverse** | **TTGCCTTGCGTGTTTGATATTC** | **40** | |
| **mGAPDH** | **Forward** | ACCCCAGCAAGGACACTGAGCAA G | **41** | **92** |
| | **Reverse** | TGGGGGTCTGGGATGGAAATTGT G | **42** | |

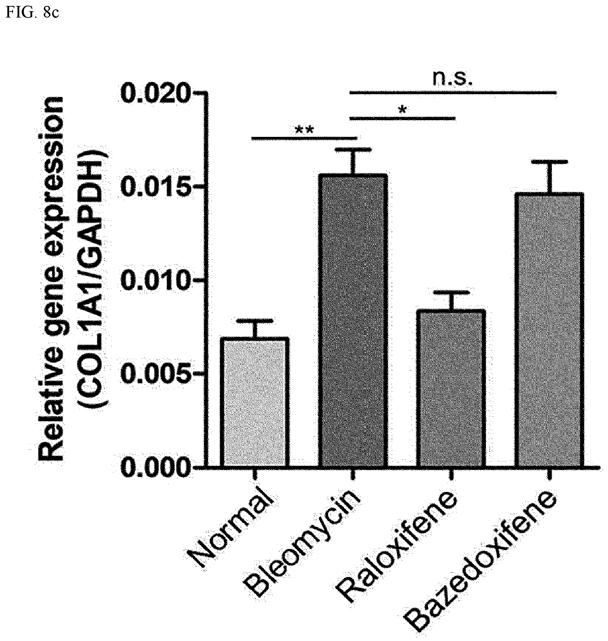

Through the above experiment, it was confirmed that the expressions of COL1A1, COL3A1, and ACTA2, which are markers of fibrosis factors, were reduced by treatment with raloxifene (FIGS. 8C to E).

### <8-3> Analysis of thickness of skin tissue by raloxifene treatment in bleomycin model

In order to verify the antifibrotic efficacy of raloxifene in the bleomycin model established in Example 8-1 above, the skin tissue was harvested and paraffin blocks were prepared. The harvested skin tissue was fixed in 4% formaldehyde. The tissue was maintained in the running water for a day to remove the remaining fixative. Dehydration was carried out sequentially while increasing the concentration of an ethanol solution. After dehydration, a transparent process was performed with xylene, followed by paraffin penetration. The next day, the tissue was fixed to a paraffin block, and a tissue section was obtained using a microtome. The slide was dried at 60°C for 1 hour. After the slide was deparaffinized with xylene, it was rehydrated while decreasing the concentration of the ethanol solution and washed with tap water, and staining was performed.

For hematoxylin and eosin (H&E) staining, sections were incubated in the Harris hematoxylin solution for 10 minutes. The slide was washed, decolorized in an ethanol solution containing 1% HCl, and then neutralized in 0.2% ammonia water. Control staining was performed with an eosin solution for 1 minute and 30 seconds. The slide was washed and subjected to a dehydration process while increasing the concentration of ethanol. After removing the remaining ethanol with xylene, it was sealed using the VectaMount Permanent Mounting Medium.

For Masson Trichrome staining, the slide was mordated overnight in the Bouin solution. After washing the slide, nuclei were stained for 10 minutes with Weigert Iron Hematoxylin. The cytoplasm was stained for 5 minutes with Biebrich scarlet-acid fuchsin. It was decolorized for 10 minutes with a solution in which phosphotungstic acid, phosphomolybdic acid, and DW were mixed at a ratio of 1:1:2. The collagenic fiber was stained by maintaining in a 2% aniline blue solution for 5 minutes. The slide was washed and subjected to a dehydration process while increasing the concentration of ethanol. The remaining ethanol was removed with xylene, and it was sealed using the VectaMount Permanent Mounting Medium.

The slide was maintained for 1 hour in a Picrosirius Red solution for Picrosirius Red staining. It was washed with acetic acid and subjected to a dehydration process while increasing the concentration of ethanol. After removing the remaining ethanol with xylene, it was sealed using the VectaMount Permanent Mounting Medium.

For immunochemical staining, it was cultured for 15 minutes in 3% hydrogen peroxide to block endogenous peroxidase. After washing the slide, the cells were blocked for 1 hour at room temperature with PBS containing 1% bovine serum albumin (BSA), and then, the primary antibody was diluted in PBA at the following dilution ratio: α-SMA (1/200; Abcam). The primary antibody was incubated at room temperature at 4°C for one day. The next day, it was washed with Tris buffered saline (TBS) containing 0.1% Tween-20 (TBST), and after the secondary antibody was maintained at room temperature for 10 minutes, it was cultured in the ABC reagent for 10 minutes. The slide was washed with PBS, and the DAB solution was maintained for 1 minute. Mayer's hematoxylin was applied for 1 minute as a nuclear staining agent. The slide was washed and subjected to a dehydration process while increasing the concentration of ethanol. The remaining ethanol was removed with xylene, and it was sealed using the VectaMount Permanent Mounting Medium.

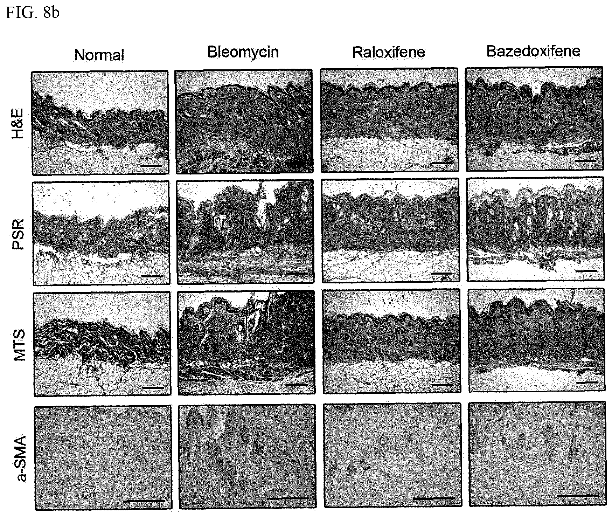

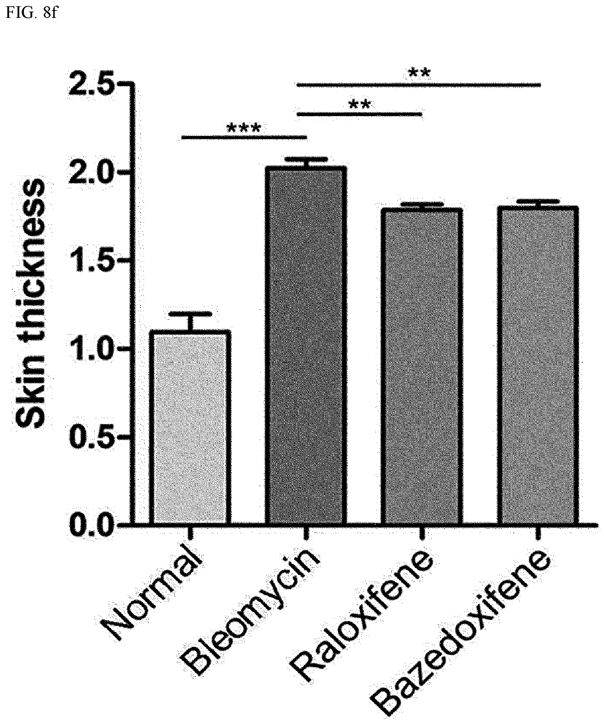

As a result, it was confirmed that the thickness of the skin tissue, which was increased in the bleomycin mouse model, was reduced by raloxifene (FIGS. 8B and F).

### <8-4> Analysis of pulmonary fibrosis by raloxifene treatment in bleomycin model

In order to confirm the effect of reducing pulmonary fibrosis by treatment with raloxifene in the bleomycin model established in Example 8-1 above, Masson Trichrome staining was performed. For Masson Trichrome staining, the slide was mordated overnight in the Bouin solution. After washing the slide, nuclei were stained for 10 minutes with Weigert Iron Hematoxylin. The cytoplasm was stained for 5 minutes with Biebrich scarlet-acid fuchsin. It was decolorized for 10 minutes with a solution in which phosphotungstic acid, phosphomolybdic acid, and DW were mixed at a ratio of 1:1:2. The collagenic fiber was stained by maintaining in a 2% aniline blue solution for 5 minutes. The slide was washed and subjected to a dehydration process while increasing the concentration of ethanol. The remaining ethanol was removed with xylene, and it was sealed using the VectaMount Permanent Mounting Medium.

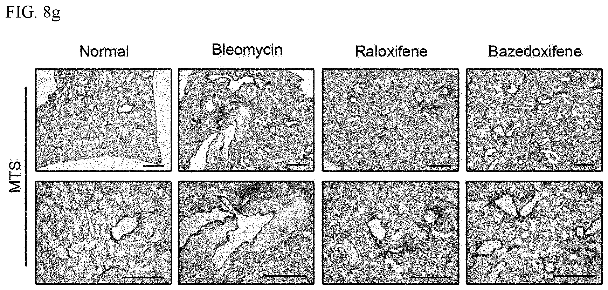

As a result, it was confirmed that lung fibrosis, which was increased in the bleomycin mouse model, was reduced by raloxifene (FIG. 8G).

### Example 9

### Verification of efficacy of fibrosis drug using dermal fibrosis model derived from induced pluripotent stem cells

### <9-1> Analysis of collagen expression

In order to verify the fibrosis model of the fibroblasts derived from the induced pluripotent stem cells of patients with systemic sclerosis formed in Example 3 above, 2.5 × 10⁵ fibroblasts were plated on a 6-well plate, and maintained and expanded for 1 day. After 1 day, pirfenidone and nintedanib, which are drugs approved as anti-fibrotic drugs, were treated at a concentration of 5 µM. Collagen expression was measured using the hydroxyproline assay kit (Sigma). The cells were harvested, mixed with hydrochloric acid at a ratio of 1:1, and incubated at 120°C for 3 hours. After incubation, only the supernatant was separated by centrifugation at 13,000 xg for 5 minutes. The harvested supernatant was transferred to a 96-well plate and dried completely at 60°C. The Chloramine T/Oxidation Buffer mixture was added to the wells and incubated at room temperature for 5 minutes. After adding the diluted DMAB Reagent and incubating at 60°C for 90 minutes, absorbance was measured at 560 nm to measure the total amount of collagen.

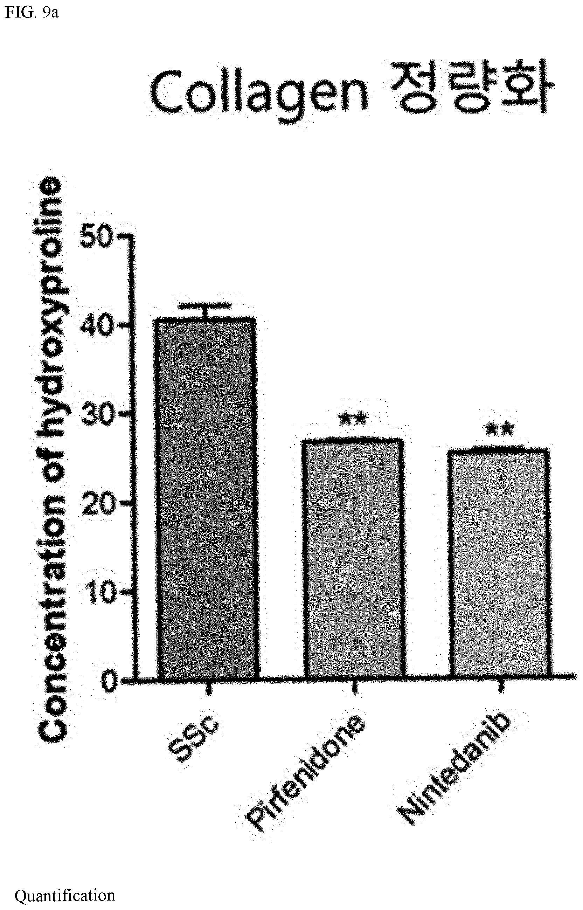

As a result, it was confirmed that the expression of the total collagen was significantly reduced in the group treated with the drugs pirfenidone and nintedanib, compared to the fibroblasts derived from the induced pluripotent stem cells of patients with systemic sclerosis, for which the drugs were not treated (FIG. 9A).

Through the above results, the drug screening platform was verified by confirming the antifibrotic effect of the previously known therapeutic agents of fibrosis in the induced pluripotent stem cell-derived fibrosis model.

### <9-2> Analysis of expression of fibrosis markers

In order to verify the fibrosis model of the fibroblasts derived from the induced pluripotent stem cells of patients with systemic sclerosis formed in Example 3 above, 2.5 × 10⁵ fibroblasts were plated on a 6-well plate, and maintained and expanded for 1 day. One day later, pirfenidone and nintedanib were treated at a concentration of 5 µM. The total RNA was extracted using Trizol, and cDNA was synthesized using the Revert Aid TM First Strand cDNA Synthesis kit. The qRT-PCR was performed on the synthesized cDNA using LightCycle 480 SYBR Green. The primers used are presented in [Table 3]. All of the experiments were repeated 3 times, and the threshold of the average cycle was used to calculate gene expression for averaging GAPDH as an internal control.

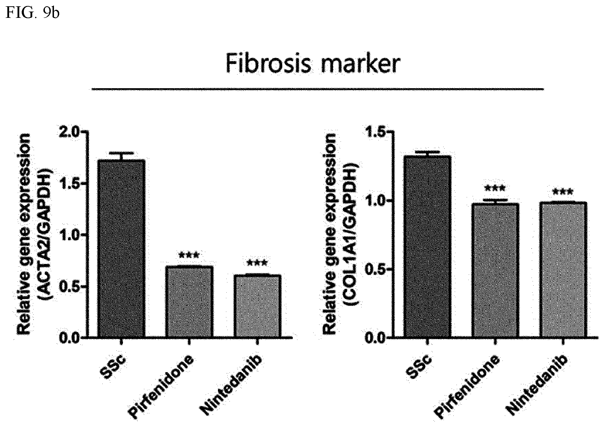

As a result, it was confirmed that the gene expressions of ACTA2 and COL1A1, which are the fibrosis factors, were significantly reduced in the group treated with pirfenidone and nintedanib, compared to the fibroblasts derived from the induced pluripotent stem cells of patients with systemic sclerosis for which the drugs were not treated (FIG. 9B).

Through the above results, the drug screening platform was verified by confirming the antifibrotic effect of the previously known therapeutic agents of fibrosis in the induced pluripotent stem cell-derived fibrosis model.

## Claims

1. A method for producing a systemic sclerosis disease model, comprising:
a step of differentiating induced pluripotent stem cells (iPSC) derived from patients with systemic sclerosis (SSc) into keratinocytes or fibroblasts; and
a step of forming a 3D skin organoid by three-dimensionally culturing the keratinocytes and/or fibroblasts,
wherein in cells of the organoid, the accumulation of collagen is increased, and the expression of α-smooth muscle actin (a-SMA) is increased, compared to normal cells.

2. A method for producing a systemic sclerosis disease model, comprising:
a step of differentiating induced pluripotent stem cells (iPSC) derived from patients with systemic sclerosis (SSc) into keratinocytes or fibroblasts;
a step of forming a 3D skin organoid by three-dimensionally culturing the keratinocytes or fibroblasts; and
a step of transplanting the 3D skin organoid into the skin tissue of a mouse,
wherein the skin thickness of the mouse transplanted with the 3D skin organoid is increased compared to normal mice.

3. A systemic sclerosis disease model, produced by the method of claim 1 or 2.

4. A method for differentiating induced pluripotent stem cells into keratinocytes, comprising:
a step of primarily culturing an embryonic body (EB) of induced pluripotent stem cells (iPSC) derived from patients with systemic sclerosis (SSc) in Differentiation Culture Medium 1 comprising DMEM/F12 for 3 to 10 days;
a step of secondarily culturing for 2 to 10 days by replacing the culture medium with Differentiation Culture Medium 2 comprising a defined keratinocyte serum-free medium (DKSFM); and
a step of tertiarily culturing for 5 to 60 days by replacing the secondary culture medium with Differentiation Culture Medium 3 comprising DKSFM and a keratinocyte serum-free medium (KSFM).

5. The method of claim 4, wherein the primary culture period is 7 to 9 days, the secondary culture period is 3 to 6 days, and the tertiary culture period is 15 to 25 days.

6. A method for differentiating induced pluripotent stem cells into fibroblasts, comprising:
a step of culturing an embryonic body (EB) of induced pluripotent stem cells (iPSC) derived from patients with systemic sclerosis (SSc) in Differentiation Culture Medium 1 comprising DMEM/F12 and epidermal growth factor (EGF) for 1 to 7 days;
a step of maintaining for 1 to 7 days by adding BMP4 to the Differentiation Culture Medium 1;
a step of secondarily culturing for 5 to 15 days by replacing the culture medium with Differentiation Culture Medium 2 comprising DMEM/F12 thereafter; and
a step of tertiarily culturing for 5 to 20 days by replacing the culture medium with Differentiation Culture Medium 1 thereafter.

7. The method of claim 6, wherein the primary culture period is 2 to 5 days, the secondary culture period is 5 to 10 days, and the tertiary culture period is 5 to 15 days.

8. The method of claim 6, comprising additional step of additionally culturing by placing the cultured cells on a collagen 1-coated plate after tertiarily culturing.

9. The method of claim 8, comprising additionally culturing for 5 days or more by placing on a collagen 1-coated plate.

10. A 3D skin organoid, comprising keratinocytes produced by the method of claim 4; and fibroblasts produced by the method of claim 6.

11. The 3D skin organoid of claim 10, wherein in cells of the organoid, the accumulation of collagen is increased, and the expression of α-smooth muscle actin (a-SMA) is increased, compared to normal cells.

12. A fibroblast derived from induced pluripotent stem cells, wherein the fibroblast is a fibroblast derived from induced pluripotent stem cells from patients with systemic sclerosis in which the expression of a fibrosis marker is increased, compared to a fibroblast derived from normal induced pluripotent stem cells, and
wherein the fibrosis marker comprises one or more selected from the group consisting of COL1A1, COL1A2, COL3A1, ACTA2, and vimentin.

13. The fibroblast of claim 12, wherein the induced pluripotent cells are reprogrammed cells from cells from patients with systemic sclerosis.

14. The fibroblast of claim 12, wherein the accumulation of collagen is increased, and the expression of α-smooth muscle actin (a-SMA) is increased, compared to fibroblasts derived from normal induced pluripotent stem cells.

15. A systemic sclerosis disease model, comprising the 3D skin organoid of claim 10 or the fibroblast of claim 12.

16. A systemic sclerosis disease model, wherein the disease model of claim 15 is a mouse.

17. A systemic sclerosis disease model, comprising a 3D cell aggregate formed by culturing induced pluripotent stem cells (iPSC) derived from patients with systemic sclerosis (SSc), wherein the cell aggregate exhibits a pathological property of systemic sclerosis, and
wherein the pathological property of cells exhibits increased cell proliferation and the increased production and accumulation of fibrosis markers and collagen, compared to normal induced pluripotent stem cells.

18. A method for screening an antifibrotic drug comprising a therapeutic agent for systemic sclerosis, the method comprising:
(a) producing a 3D skin organoid by differentiating induced pluripotent stem cells (iPSC) derived from patients with systemic sclerosis (SSc) into keratinocytes or fibroblasts to three-dimensionally culture the keratinocytes and fibroblasts;
(b) treating the 3D skin organoid with a test agent that inhibits collagen accumulation;
(c) measuring the expression level of α-smooth muscle actin (a-SMA) or mRNA of a gene thereof in the 3D skin organoid treated with the test agent; and
(d) determining the test agent as a therapeutic agent for systemic sclerosis, when the expression level of α-SMA or mRNA of a gene thereof measured in Step (c) is decreased, compared to a 3D skin organoid not treated with a test agent.

19. A method for screening an antifibrotic drug comprising a therapeutic agent for systemic sclerosis, the method comprising:
(a) producing a 3D skin organoid by differentiating induced pluripotent stem cells (iPSC) derived from patients with systemic sclerosis (SSc) into keratinocytes or fibroblasts to three-dimensionally culture the keratinocytes and fibroblasts;
(b) treating the 3D skin organoid with a test agent that inhibits collagen accumulation;
(c) measuring the level of collagen accumulation in the 3D skin organoid treated with the test agent; and
(d) determining the test agent as a therapeutic agent for systemic sclerosis, when the level of collagen accumulation measured in Step (c) is decreased, compared to a 3D skin organoid not treated with a test agent.

20. A method for screening an antifibrotic drug comprising a therapeutic agent for systemic sclerosis, the method comprising:
(a) differentiating induced pluripotent stem cells (iPSC) derived from patients with systemic sclerosis (SSc) into fibroblasts;
(b) treating the fibroblasts differentiated in Step (a) with a test agent that inhibits collagen accumulation;
(c) measuring the expression level of α-smooth muscle actin (a-SMA) or mRNA of a gene thereof in the fibroblasts treated with the test agent; and
(d) determining the test agent as a therapeutic agent for systemic sclerosis, when the expression level of α-SMA or mRNA of a gene thereof measured in Step (c) is decreased, compared to fibroblasts not treated with a test agent.

21. A method for screening an antifibrotic drug comprising a therapeutic agent for systemic sclerosis, the method comprising:
(a) differentiating induced pluripotent stem cells (iPSC) derived from patients with systemic sclerosis (SSc) into fibroblasts;
(b) treating the fibroblasts differentiated in Step (a) with a test agent that inhibits collagen accumulation;
(c) measuring the level of collagen accumulation in the fibroblasts treated with the test agent; and
(d) determining the test agent as a therapeutic agent for systemic sclerosis, when the level of collagen accumulation measured in Step (c) is decreased, compared to fibroblasts not treated with a test agent.

22. An antifibrotic drug comprising the therapeutic agent for systemic sclerosis screened by any one of claims 18 to 21.
